(19) **Europäisches Patentamt / European Patent Office / Office européen des brevets**

(11) **EP 1 430 014 B1**

(12) **EUROPÄISCHE PATENTSCHRIFT**

(45) Veröffentlichungstag und Bekanntmachung des Hinweises auf die Patenterteilung:
**07.11.2007 Patentblatt 2007/45**

(21) Anmeldenummer: **02762477.4**

(22) Anmeldetag: **20.09.2002**

(51) Int Cl.:
*C07C 67/08* (2006.01)    *C07C 69/80* (2006.01)
*C08K 5/10* (2006.01)    *C10M 159/12* (2006.01)

(86) Internationale Anmeldenummer:
**PCT/EP2002/010570**

(87) Internationale Veröffentlichungsnummer:
**WO 2003/029180 (10.04.2003 Gazette 2003/15)**

(54) **PHTHALSÄUREALKYLESTERGEMISCHE MIT KONTROLLIERTER VISKOSITÄT**

PHTHALIC ACID ALKYLESTER MIXTURES WITH CONTROLLED VISCOSITY

MELANGES D'ALKYLESTERS D'ACIDE PHTALIQUE DE VISCOSITE CONTROLEE

(84) Benannte Vertragsstaaten:
**AT BE BG CH CY CZ DE DK EE ES FI FR GB GR IE IT LI LU MC NL PT SE SK TR**

(30) Priorität: **26.09.2001 DE 10147567**
**16.01.2002 DE 10201348**

(43) Veröffentlichungstag der Anmeldung:
**23.06.2004 Patentblatt 2004/26**

(73) Patentinhaber: **Oxeno Olefinchemie GmbH 45772 Marl (DE)**

(72) Erfinder:
• **WIESE, Klaus-Diether**
**45721 Haltern am See (DE)**

• **GRASS, Michael**
**45721 Haltern am See (DE)**
• **BÜSCHKEN, Wilfried**
**45721 Haltern am See (DE)**

(74) Vertreter: **Hirsch, Hans-Ludwig et al Degussa GmbH Intellectual Property Management PATENTE & MARKEN Bau 1042 / PB 15 45764 Marl (DE)**

(56) Entgegenhaltungen:
**EP-A- 0 424 767      EP-A- 1 029 839**
**WO-A-00/63151      DE-A- 19 918 051**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Gegenstand der Erfindung sind Gemische von Phthalsäureestern mit einer über die Beiträge der einzelnen isomeren Alkohole, aus dem die Phthalsäureester hergestellt werden können, einstellbaren Viskosität und deren Verwendung.

## Hintergrund

Phthalsäureester werden in großem Umfang als Weichmacher für Kunststoffe wie PVC eingesetzt. Weichmacher können definiert werden als Substanzen, die einem Material, insbesondere Kunststoffen, erhöhte Flexibilität, Weichheit und Verarbeitbarkeit verleihen (Alan S. Wilson, Plasticizers, The Institute of Materials, 1995, ISBN 0 901716 76 6, S. 1). Insbesonders, aber nicht ausschließlich, ist die Weichmachung von PVC von besonderer Bedeutung.

Seit über 100 Jahren sind immer wieder neue Weichmacher entwickelt worden, wobei mit wenigen Ausnahmen mehrwertige Ester eingesetzt werden. Am häufigsten werden Ester aus mehrwertigen Carbonsäuren und Monoalkoholen verwendet. Beispiele für eingesetzte mehrwertige aromatische Carbonsäuren oder ihre Anhydride sind Phthalsäure und Isophthalsäure, Trimellitsäure, Pyromellitsäure oder Terephthalsäure. Auch anorganische Säuren werden verwendet, bekanntestes Beispiel sind die Ester der Phosphorsäure. In der Regel werden diese Carbonsäuren oder ihre Anhydride, im Falle der Phosphorsäureester auch die Säurechloride wie $POCl_3$ umgesetzt mit Monoalkoholen wie Ethanol, Butanol, Isobutanol, n- und iso-Amylalkoholen, Heptanol, 2-Ethylhexanol, 2-Propylheptanol. Als höhere Alkohole werden bevorzugt auch Isomerengemische eingesetzt, die erhalten werden durch Oligomerisierung von Olefinen mit 3 bis 5 C-Atomen mit nachfolgender Hydroformylierung und Hydrierung der erhaltenen Aldehyde. Technische Beispiele sind Isoheptanol, Isooctanol, Isononanol, Isodecanol, Isotridecanol. Durch Oligomerisierung von Ethylen sind auch lineare sogenannte Alphaolefine erhältlich, die bei der Hydroformylierung ein Gemisch aus linearen und wenig verzweigten Alkoholen ergeben. Beispiele für so technisch hergestellte Alkohole sind Nonanol und Undecanol und deren Mischungen. Geradkettige Alkohole sind aus der Fettchemie zugänglich, aber auch durch synthetischen Aufbau aus Ethylen, z.B. die sogenannten Ziegler-Alkohole. Schließlich seien noch cyclische Alkohole erwähnt wie Cyclohexanol, Benzylalkohol, Phenole, Cresole, Xylole, die technische Verwendung gefunden haben.

An Stelle mehrwertiger Carbonsäuren und Monoalkoholen kann man auch umgekehrt mehrwertige Alkohole mit Monocarbonsäuren zu Weichmachern umsetzen. Beispiele für mehrwertige Alkohole sind Neopentylglycol, Trimethylolpropan, Pentaerythrit, DiPentaerythrit, Ethylenglycol und seine Oligomeren Di-, Tri- und Tetraethylenglycol, Glycerin, Butandiol, Hexandiol und so weiter. Auch Zuckeralkohole sind schon zu diesem Zweck eingesetzt worden. Als Carbonsäuren werden hier die analogen Verbindungen zu den oben genannten Alkoholen eingesetzt, also Carbonsäuren im Bereich von 2 bis etwa 13 C-Atomen. Beispiele sind die Essigsäure im Triacetin, Buttersäuren, Valeriansäuren, Heptan- und Nonansäure sowie Isomerengemische wie Isoheptan-, Isooctan-, Isononan-, Isodecan- und Isotridecansäure.

Auch Verbindungen, die sowohl Säure- wie Alkoholfunktion enthalten, sind einsetzbar. Bekanntestes Beispiel ist die Zitronensäure mit drei Carbonsäuren- und einer Alkoholgruppe. Durch Veresterung der Carbonsäuregruppen mit Monoalkoholen erhält man brauchbare Ester, die Alkoholgruppe kann dann zusätzlich mit einer Carbonsäure wie beispielsweise Buttersäure oder Essigsäure verestert werden.

Der Vollständigkeit halber sei erwähnt, dass auch Ester von Monoalkoholen und Monocarbonsäuren in Sonderfällen als Weichmacher verwendet werden, wenn ihr Siedepunkt hoch genug ist, wie Stearinsäure- oder Laurinsäureester.

Auch die vorstehende Aufzählung ist nicht vollständig. So sind auch Kombinationen mehrwertiger Alkohole mit mehrwertigen Carbonsäuren schon als Weichmacher eingesetzt worden. Zum Beispiel kann man Diole und Dicarbonsäuren zu niedermolekularen Polyestern verknüpfen, deren Endgruppen man dann mit funktionell einwertigen Alkoholen bzw. Carbonsäuren verestert. Die restlichen Alkoholgruppen werden wieder mit Monocarbonsäuren verestert. Auch Etherbindungen können genutzt werden, um zu einer größeren Anzahl veresterbarer Gruppen zu kommen, wie im Di- und Tri-Pentaerythrit mit 6 bzw. 8 veresterbaren Alkoholgruppen. Dies wird insbesondere dann gemacht, wenn besonders hochmolekulare Weichmacher mit hohem polaren Anteil erwünscht sind.

Ziel ist in aller Regel, Verbindungen herzustellen, die ausreichende Polarität haben, um als gute Weichmacher zu dienen, die eine hohe Molmasse haben, um möglichst wenig flüchtig zu sein und geringe Migration im Material zu zeigen, aber dennoch flüssig sind und eine im Vergleich zur Molmasse geringe Viskosität aufweisen, sodass sie gut verarbeitbar sind.

Erwähnt werden soll noch, dass Verbindungen oder Gemische von Verbindungen, die diese Eigenschaften aufweisen, auch wichtige andere Einsatzgebiete haben, zum Beispiel als synthetische Schmierstoffe, Hydrauliköle, aber auch als Lösungsmittel in Salben, Tinten usw.. Man spricht von Funktionsflüssigkeiten ("functional fluids").

Auf die zuvor beschriebene Weise lassen sich für nahezu jeden Anwendungsfall passende Ester maßschneidern, wobei natürlich Grenzen gesetzt sind durch den erforderlichen Aufwand und die hiermit verbundenen Kosten sowie durch die Verfügbarkeit der Rohstoffe. Viele der aufgeführten möglichen Kombinationen sind deshalb heute vom Markt verschwunden, haben nur begrenzte Bedeutung in Nischenanwendungen gefunden oder sind nur von akademischer Bedeutung. Dies kann sich aber unter Umständen schnell ändern, wenn durch neue Verfahren Rohstoffe preiswert zugänglich werden, durch verändertes Umweltbewußtsein Ersatzstoffe für bisher gängige Produkte gefunden werden müssen oder neue technische Anforderungen zu neuen Lösungen zwingen.

Die folgenden Ausführungen beschränken sich ausschließlich der Übersichtlichkeit halber auf wenige ausgewählte technisch besonders relevante Beispiele aus dem Anwendungsbereich der Weichmacher für PVC. Grundsätzlich ist die im Folgenden beschriebene Problematik und ihre Lösung aber übertragbar auf alle genannten Ester.

## Problemstellung

Die mit Abstand wichtigste Esterklasse für Weichmacher sind die Phthalate. Unter diesen wiederum ist heute noch das Phthalat des 2-Ethylhexanols (oft auch verkürzt "Octanol" genannt) vorherrschend, das DOP (Dioctylphthalat) oder DEHP (Diethylhexylphthalat). Zunehmend wird es aber verdrängt durch DINP (Diisononylphthalat), das in seinen anwendungstechnischen Eigenschaften deutliche Vorteile aufweist wie geringere Flüchtigkeit und geringere Migration. Im Gegensatz zu DEHP, das, abgesehen von Stereoisomeren, eine chemisch eindeutig definierte Substanz ist mit eindeutig bestimmbaren Eigenschaften, ist DINP ein Gemisch vieler ähnlicher Isomerer mit gleicher Molmasse. Dies beruht auf der Herkunft des zur Veresterung eingesetzten Isononanols.

Isononanol wird hergestellt durch Hydroformylierung von Octenen, die wiederum auf unterschiedliche Art erzeugt werden. Als Rohstoff dienen im Allgemeinen technische $C_4$-Ströme, die zunächst alle isomeren $C_4$-Olefine enthalten neben den gesättigten Butanen und ggf. Verunreinigungen wie $C_3$- und $C_5$-Olefinen und acetylenischen Verbindungen. Butadien wird zunächst als Wertstoff gewonnen durch Extraktivdestillation, zum Beispiel mit NMP (N-Methylpyrrolidon). Alternativ kann das Butadien auch durch selektive Hydrierung in 1- und 2-Buten überführt werden, wie z. B. im SHP-CB-Prozess der OXENO GmbH. In beiden Fällen erhält man einen $C_4$-Strom, der an Olefinen im Wesentlichen nur noch Isobuten, 1- und 2-Buten enthält, häufig als "Raffinat I" bezeichnet. Durch Oligomerisierung dieses Olefingemisches erhält man vorwiegend Octene neben höheren Oligomeren wie $C_{12}$- und $C_{16}$-Olefingemischen. Bei dem $C_8$-Schnitt spricht man von "Codibutylen", das früher zur Herstellung von Isononanolen für Weichmacher eingesetzt wurde.

Heute wird das Isobuten in der Regel in einem zweiten Verfahrensschritt entfernt durch Reaktion mit Methanol unter saurer Katalyse, zum Beispiel mit sauren Ionenaustauschern. Man erhält dabei den als Kraftstoffzusatz wichtigen Methyl-t-butyl-ether (MTBE) und einen weitgehend isobutenfreien $C_4$-Strom, das sogenannte "Raffinat II". An Stelle von Methanol können auch andere Alkohole eingesetzt werden wie Ethanol. In diesem Fall erhält man den Ethyl-t-butyl-ether, das ETBE. Auch mit Wasser an Stelle von Alkoholen ist die selektive Entfernung von Isobuten möglich unter Bildung des t-Butylalkohols TBA, der ebenfalls eine wichtige technisch genutzte Verbindung ist. Die Rückspaltung des TBA führt zu hochreinem Isobuten. Bei allen diskutierten Varianten bleibt ein $C_4$-Strom übrig, der im Wesentlichen nur noch 1- und 2-Buten enthält neben den gesättigten Butanen, das sogenannte "Raffinat II".

Aus dem Raffinat II kann optional 1-Buten als Wertstoff gewonnen werden, zum Beispiel als Comonomeres für Polyolefine, ebenso Isobutan, das Verwendung findet als Treibgas. Man erhält dann einen an 1-Buten abgereicherten C4-Strom, das Raffinat III. Sowohl Raffinat II als auch Raffinat III können als Rohstoff eingesetzt werden für die Oligomerisierung. Hier gibt es wieder verschiedene technisch genutzte Möglichkeiten. Die älteren Oligomerisierungsverfahren arbeiten mit sauren Katalysatoren wie Phosphorsäure auf Trägern oder sauren Zeolithen (Verfahrensvariante A). Hierbei erhält man Octene, die im Wesentlichen Dimethylhexene enthalten. Neuere Verfahren wie der DIMERSOL-Prozess arbeiten mit löslichen Ni-KomplexKatalysatoren. Hier werden Octengemische erhalten mit hohen Anteilen an 3- und 5-Methylheptenen neben n-Octenen und Dimethylhexenen (Verfahrensvariante B). Die modernsten Verfahren nutzen die hohe Selektivität von speziellen geträgerten Ni-Katalysatoren, bekannt ist der OCTOL-Prozess der OXENO GmbH. Hiermit werden die mit am wenigsten verzweigten Octengemische erzielt, was für den Einsatz für Weichmacheralkohole besonders günstig ist (Verfahrensvariante C). Die folgende Tabelle gibt nur ungefähre Werte für die Zusammensetzung der jeweils erhaltenen Produkte an, da die genaue Zusammensetzung vom eingesetzten Katalysator, Temperatur, Verweilzeit, Umsatzgrad und anderen Bedingungen abhängt. Es ist aber deutlich zu erkennen, dass je nach Verfahren unterschiedliche Isomerenzusammensetzungen erhalten werden. Aus Raffinat II oder III erhält man

|  | A saure Katalyse | B Dimersol | C Octol |
|---|---|---|---|
| n-Octene | 0 % | 6 % | 13 % |
| Methyl-Heptene | 5 % | 59 % | 62 % |
| Dimethyl-Hexene | 70 % | 34 % | 24 % |

(fortgesetzt)

|  | A saure Katalyse | B Dimersol | C Octol |
|---|---|---|---|
| Sonstige | 25 % | 1 % | 1 % |

Eine weitere Möglichkeiten, zu Octenen zu kommen, ist die Oligomerisierung von Isobuten. Das Hauptprodukt ist dabei ein Gemisch von 2,4,4-Trimethylpenten-1 und 2,4,4-Trimethylpenten-2. Auch die Oligomerisierung von Ethylen unter Ziegler-Katalyse oder nach dem SHOP-Prozess ist ein Weg zu höheren Olefinen, wobei lineare endständige Olefine gebildet werden mit einer breiten C-Zahl-Verteilung. Hieraus lässt sich unter anderem ein Cg-Schnitt gewinnen. Solche Schnitte stehen auch aus Fischer-Tropsch-Verfahren zur Verfügung. Schließlich wird auch gelegentlich die Möglichkeit genutzt, aus Naphthaströmen Kohlenwasserstoffe geeigneter C-Zahl abzutrennen. Diese enthalten vorwiegend gesättigte Alkane, die durch Dehydrierung in Olefine überführt werden.

Eine ganz andere Möglichkeit ist die Oligomerisierung von rohen nicht aufgetrennten Olefinströmen, wie sie bei der Aufarbeitung in Crackern entstehen, z.B. Schnitte, die $C_3$-, $C_4$- und gegebenenfalls $C_5$-Olefine enthalten. Solche Ströme liefern sauer oligomerisiert ein Gemisch aller denkbaren Kombinationen dieser Olefine, also Hexene, Heptene, Octene, Nonene, Decene usw., die in C-Zahl-Schnitte zerlegt werden, sogenannte Polygas-Olefine. Man erhält hier vorwiegend höher verzweigte innenständige Olefine.

Insgesamt gibt es eine Vielzahl von Verfahren, die Olefine geeigneter Kettenlänge liefern für die Oxierung/ Hydrierung zu Weichmacheralkoholen. Je nach Verfahren werden Isomerengemische unterschiedlichsten Verzweigungsgrades erhalten - von unverzweigt bis 3- und mehrfach verzweigt, und mit unterschiedlicher Verteilung der Doppelbindung von endständig bis fast ausschließlich innenständig.

An die Oligomerisierung schließt sich als nächster Verfahrensschritt eine Hydroformylierung an, also die Umsetzung der Olefine mit Kohlenmonoxid und Wasserstoff, dem sogenannten Synthesegas, zu Aldehyden. Auch hierbei werden technisch mehrere Varianten eingesetzt. Gängig ist die Hydroformylierung mit Hydridocobaltcarbonyl als Katalysator bei 200 bis 300 bar und 170 bis 190 °C (Co-Hochdruck-Verfahren Co-HD). Auch der Einsatz von mit Alkylphosphinen modifizierten Co-Katalysatoren wird technisch ausgeübt (Co-Ligand). Des weiteren ist ein Verfahren bekannt, das mit Rhodium an Stelle von Cobalt als Katalysator arbeitet bei 100 bis 300 bar und 120 bis 130°C (Rh-HD). Die drei Verfahren unterscheiden sich deutlich in der Isomerenzusammensetzung der Produkte. Zum Beispiel werden bei der Oxierung von innenständigen linearen Octenen bei Co-HD-Oxierung ca. 50 % lineares n-Nonanol erhalten neben den innenständig oxierten Isomeren (n/i ~ 1), bei der Oxierung mit ligandmodifiziertem Co ca. 80 % (n/i ~ 4), bei Rh-HD-Oxierung ca. 20 % (n/i ~ 0,25). Ähnliches gilt auch für die Oxierung der verzweigten Olefine.

Es bleibt festzuhalten, dass selbst bei gleicher Summenformel, im Beispiel $C_8H_{16}$ für Octene, die unterschiedlichsten Struktur- und Doppelbindungsisomeren in den technisch eingesetzten Olefinschnitten vorhanden sind. Selbst bei Einsatz eines identischen Olefinschnittes ist der Verzweigungsgrad der über Oxierung/Hydrierung erhaltenen Weichmacheralkohole sehr unterschiedlich in Abhängigkeit vom eingesetzten Oxierungsverfahren. Und auch bei einer festen Kombination eines Oligomerisierungsverfahrens mit einem Oxierungsverfahren treten Schwankungen in der Isomerenzusammensetzung auf durch Schwankungen in der Zusammensetzung der eingesetzten rohen Ausgangsstoffe wie $C_4$-Schnitt, durch Änderungen der Betriebsbedingungen, durch Anpassung des Umsatzes an die erforderliche Produktion und vieles mehr.

Die Isomerenzusammensetzung der erzeugten Weichmacheralkohole kann somit in weiten Grenzen schwanken. Es ist aber bekannt, dass die physikalischen und anwendungstechnischen Eigenschaften von Weichmachern stark abhängig sind von der Struktur. Im Folgenden ist als Beispiel einer physikalischen Eigenschaft die Viskosität der Phthalate, gemessen bei 20 °C, angegeben. So können Phthalate von Isononanol (DINP), Viskositäten aufweisen im Bereich von 50 bis 60 mPa*s bei Einsatz linearer Olefine, bekannt sind aber auch DINP-Typen mit 160 bis 170 mPa*s bei Einsatz von Codibutylen. DINP-Typen auf Basis Polygas-Octenen haben Viskositäten im Bereich 90 bis 110 mPa*s. Bei Einsatz von Raffinat II oder III erhält man mit dem Octol-Verfahren Alkohole, deren Phthalate Viskositäten im Bereich von 72 bis 82 mPa*s haben, wenn sie mit Co-Hochdruckverfahren oxiert werden. Mit Rh-Katalysatoren führt das gleiche Edukt zu Weichmachern mit 90 bis 100 mPa*s.

Die folgenden Ausführungen beschränken sich im Wesentlichen auf einige Varianten von Weichmachern auf Basis von Octenen, es ist dem Fachmann leicht verständlich, dass die Problematik immer vorhanden ist, wenn Gemische und insbesondere Isomerengemische zur Herstellung von Estern als Funktionsflüssigkeiten eingesetzt werden, wobei hier Weichmacher als Untergruppe von Funktionsflüssigkeiten betrachtet werden.

Die anwendungstechnischen Eigenschaften, die notwendig sind, dass ein Ester seine Funktion erfüllt, sind ausgesprochen vielfältig. Beim Einsatz als Schmiermittel spielen zum Beispiel die Viskosität, die Abhängigkeit der Viskosität von der Temperatur, der Tropfpunkt und vieles mehr eine Rolle. Bei Weichmachern sind es neben der flexibilisierenden Wirkung, insbesondere bei niedrigen Temperaturen, ebenfalls die Viskosität des Weichmachers, aber auch bei Einsatz in Kabelummantelungen elektrische Eigenschaften. Alle diese Eigenschaften sind abhängig von der Struktur und damit von der Isomerenzusammensetzung der eingesetzten Stoffe.

Für die Abhängigkeit der anwendungstechnischen Eigenschaften von der Struktur gibt es qualitative Regeln. In Alan S. Wilson, Plasticizers, The Institute of Materials, 1995, ISBN 0 901716 76 6, Seiten 135-136 werden beispielsweise die Eigenschaften von Phthalaten in Abhängigkeit von Gesamt-C-Zahl, das heißt von der Molmasse, und dem Verzweigungsgrad, also unter anderem von der Isomerenzusammensetzung, diskutiert. Bei gleicher Molmasse bewirkt zum Beispiel wachsende Verzweigung unter anderem

Negative Effekte

- wachsende Viskosität
- ansteigender Dampfdruck, dadurch höhere Flüchtigkeit
- geringere Weichmachung
- geringere Thermo- und Lichtstabilität

Positive Effekte

- bessere PVC-Verträglichkeit
- geringere Migration
- größere Hydrolysebeständigkeit
- geringerer biologischer Abbau (während der Gebrauchsphase)
- höherer elektrischer Widerstand

Es ist sofort ersichtlich, dass es den besten Weichmacher nicht gibt, man muss je nach Anwendung einen Kompromiss schließen. So wird man, wenn die weichmachende Wirkung im Vordergrund steht, einen Weichmacher mit möglichst geringer Verzweigung bevorzugen. Will man hingegen mit PVC Kabelummantelungen herstellen, wird man eher zu Produkten mit etwas höherer Verzweigung greifen wegen der besseren elektrischen Isolierwirkung.

In Brian L. Wadey, Lucien Thil, Mo A. Khuddus, Hans Reich; The Nonyl Phthalate Ester and It's Use in flexible PVC, Journal of Vinyl Technology, 1990,12, (4), S. 208-211 wird an Hand von einigen synthetisch hergestellten Einzelisomeren an DINP sehr deutlich gezeigt, wie wichtige Eigenschaften von der Struktur der Ester abhängen.

Zusammenfassend kann festgestellt werden, dass die anwendungstechnischen Eigenschaften von der Struktur des Phthalats, diese teilweise von der Struktur des Alkohols und diese wiederum teilweise von der Struktur des zugrundeliegenden Olefins abhängen. Zusätzlich komplizierend wirkt, dass diese Verbindungen als Isomerengemisch produziert werden. Es wäre daher wünschenswert, die Gemischzusammensetzung unter Erhalt eines Gemisches mit vordefinierten Eigenschaften zu verändern bzw. zusammenzustellen.

Die große Schwierigkeit besteht nun darin, dass zur Beurteilung der anwendungstechnischen Eignung der Einzelsubstanzen eine Vielzahl von Untersuchungen notwendig sind. Bei Phthalaten als Weichmacher für PVC als einfaches Beispiel muss zunächst aus dem zur Verfügung stehenden Alkoholgemisch eine repräsentative Phthalatprobe im Labor hergestellt werden. Anschließend müssen Prüfplatten mit PVC hergestellt werden, oft mit mehreren Konzentrationen an Weichmacher, an denen die eigentlichen standardisierten Prüfungen erfolgen. Dies ist sehr aufwendig und dauert Tage bis Wochen. Beim Einsatz als Schmiermittel kann der Aufwand noch viel höher und teurer sein, beispielsweise wenn Motorentests über Wochen gefahren werden müssen.

Wenn einmal eine Rezeptur entwickelt ist, muss nun in der Produktion sichergestellt werden, dass ein Produkt mit gleichbleibenden Eigenschaften hergestellt wird. Beispielsweise darf bei Schmiermitteln die Viskosität nur in engen Grenzen variieren, um die gewünschte Viskositätsklasse einzuhalten. Bei Weichmachern muss beispielsweise die weichmachende Wirkung konstant bleiben, um die Verarbeiter des Weichmachers nicht zu ständiger Rezepturanpassung zu zwingen, oder es darf ein Grenzwert für den elektrischen Widerstand nicht unterschritten werden. Eine laufende Produktionskontrolle durch anwendungstechnische Prüfungen ist aber zeitlich gar nicht möglich, wie oben erläutert, ganz abgesehen von den Kosten, die dies verursachen würde.

Ein exaktes Konstanthalten der Isomerenzusammensetzung könnte das Problem theoretisch zwar lösen, dies ist aber in der Praxis nicht durchzuhalten. Ein Cracker wird nicht betrieben, um einen $C_4$-Strom konstanter Zusammensetzung zu liefern, sondern um Ethylen, Propylen oder Benzin oder andere Massenprodukte herzustellen. In der Praxis wird die Zusammensetzung des $C_4$-Stromes, der aus dem Cracker zur Verfügung steht, immer je nach Zusammensetzung seines Rohstoffes und nach Fahrweise schwanken. Das Problem verschärft sich weiter, wenn $C_4$-Ströme aus unterschiedlichen Crackern zugekauft werden, wie dies bei einer Großproduktion die Regel ist.

Allein schon durch den am Anfang stehenden Rohstoff kommt es somit zwangsweise zu Änderungen in der Zusammensetzung des Produktes, somit zu Änderungen in der Isomerenverteilung und damit zu Änderungen in den anwendungstechnischen Eigenschaften. Wie schon vorher diskutiert, kommen weitere Änderungen in der Produktzusammensetzung in den nachfolgenden Schritten Oligomerisierung und Hydroformylierung, zum Beispiel durch Änderung der Betriebsbedingungen oder Alterung des Oligomerisierungskatalysators, zum Tragen. Am kritischsten ist das Problem bei einem unabhängigen Esterhersteller, der die eingesetzten Alkohole zukauft, da die Produkte aus den unterschiedlichsten Oligomerisierungs- und Oxierungsverfahren stammen können. Es sei daran erinnert, dass es DINP-Typen gibt mit Viskositäten von ~ 50 bis 170 mPa*s bei 20 °C, je nach eingesetztem Rohstoff, Oligomerisierungs- und Hydroformylierungsverfahren bei der Herstellung des als Alkohol in der Veresterung eingesetzten Isononanols. Das isolierte Beispiel DINP zeigt die grundsätzliche Problematik auf Bei allen als Funktionsflüssigkeiten eingesetzten Estern gilt das natürlich entsprechend, insbesondere wenn Isomerengemische an Alkoholen oder Carbonsäuren eingesetzt werden. Es ist zwar grundsätzlich möglich, über Änderungen der Isomerenzusammensetzung Produkte maßzuschneidern, um unterschiedlichen Anwendungsbereichen gerecht zu werden, wie zuvor diskutiert. Andererseits wird man aber aus wirtschaftlichen Gründen bestrebt sein, kontinuierliche Anlagen zu betreiben. Dabei ist es sehr zeit- und kostenintensiv, wenn in kurzen Abständen die Produktion auf unterschiedliche Produktvarianten umgestellt werden muss, gar nicht zu reden von dem Problem, die gewünschten Produkteigenschaften von vornherein berücksichtigen zu müssen. Man wird also versuchen, ein Standardprodukt mit möglichst gleichbleibenden Eigenschaften herzustellen, das einen möglichst weiten Anwendungsbereich abdeckt. Andererseits ist ein Anwachsen der Kundenansprüche zu verzeichnen, und die zunehmende Kundenorientierung erfordert ein Eingehen auf Kundenwünsche, was nur durch Herstellung von Spezialprodukten neben dem Standardprodukt möglich ist.

**Aufgabenstellung**

Es besteht somit die Aufgabe, Phthalatgemische bestimmter Eigenschaften aus Alkoholgemischen verschiedenster Zusammensetzung herzustellen, wobei die Eigenschaften der Phthalate mit einfachen Mitteln über die Zusammensetzung der Alkohole steuerbar sein sollte.

Im Idealfall ist ein Verfahren zu finden, das schon während der Produktion der kritischen Vorprodukte, z. B. bei der Hydroformylierung von Olefin-Isomerengemische erzeugten Aldehyde, erlaubt, die anwendungstechnischen Eigenschaften der Endprodukte (Phthalatgemische) vorauszusagen. Dies sollte ohne langwierige anwendungstechnische Prüfungen am Endprodukt (zum Beispiel Herstellung der Alkohole durch Hydrierung bzw. der Carbonsäuren durch Oxidation, Herstellung der Ester, Erzeugung von Prüfplatten mit weichgemachtem PVC usw.) möglich sein.

**Problemlösung**

Die Viskosität der Phthalat-Ester ist von der Struktur, bzw. von der Isomerenzusammensetzung der zur Veresterung eingesetzten Alkohole abhängig. Hiermit korrelieren anwendungstechnische Größen wie zum Beispiel kälteflexibilisierende Wirkung und elektrischer Widerstand. Dies läßt sich, wenn genügend Messungen vorliegen, durch theoretisch fundierte oder auch einfache empirische Gleichungen beschreiben.

Über die besonders einfache Messung der Viskosität der einzelnen Phthalsäureester kann somit eine Aussage über andere anwendungstechnische Eigenschaften gemacht werden. Problem ist allerdings, dass dies erst am fertigen Ester, im Beispiel also am DINP, gemessen werden kann. Eine Einflußnahme auf die Alkoholzusammensetzung ist dann nicht mehr möglich.

Grundsätzlich kann die Viskosität von Gemischen isomerer Verbindungen nach einer einfachen Mischungsregel aus den Einzelkomponenten abgeschätzt werden, es gilt nach VDI-Wärmeatlas, VDI Verlag, siebte erweiterte Auflage, 194, Abschnitt Da 30

$$\ln(\eta) = \sum x_e * \ln(\eta_e)$$

mit

$\eta$     Viskosität des Gemisches
$\eta_e$    Viskosität der Einzelkomponenten ( Phthalate)
$x_e$    Molenbruch der Einzelkomponente (Phthalate)

Somit wären die Bestimmung der Viskosität der Einzelkomponenten (Phthalate) und die Messung der Isomerenzusammensetzung (Phthalate) ein geeignetes Mittel, um die Konstanthaltung der Eigenschaften des Produkts zu kontrollieren. Dies ist aber praktisch nicht möglich wegen der riesigen Anzahl an Isomeren, deren Synthese oder Abtrennung aus Reaktionsgemischen sowie deren Vermessung mit den heute zur Verfügung stehenden Mitteln nicht mit vertretbarem Aufwand durchgeführt werden kann.

Die folgende Aufstellung gibt die wichtigsten Isomeren eines zur Herstellung von DINP eingesetzten Isononanols wieder, das hergestellt wurde durch Oxierung eines Octengemisches, das seinerseits hergestellt wurde durch Oligomerisierung von 2-Buten. Mit 14 Isomeren, ohne Nennung von Stereoisomeren, kann die wesentliche Zusammensetzung wie folgt angegeben werden: n-Nonanol, 2-Methyl-octanol, 2-Ethyl-heptanol, 2-Propyl-hexanol, 4-Methyl-octanol, 3-Ethyl-heptanol, 6-Methyl-octanol, 2,3-Dimethyl-heptanol, 2-Propyl-3-Methyl-pentanol, 2-Ethyl-4-Methyl-hexanol, 2,5-Dimethyl-heptanol, 4,5-Dimethyl-heptanol, 2,3,4-Trimethyl-hexanol und 2-Ethyl-3-Methyl-hexanol.

Enthält der ursprünglich eingesetzte Rohstoff beispielsweise noch Isobuten, kommen viele weitere Isomere hinzu wie 3,5,5-Trimethyl-hexanol, 3,4,5-Trimethyl-hexanol und 3,4,4-Trimethylhexanol und andere.

Bei der Veresterung von Alkoholen mit einer zweiwertigen Polycarbonsäure, wie beispielsweise Phthalsäure, entsteht nur dann eine einzige Verbindung, wenn der eingesetzte Alkohol aus einem einzigen Isomeren besteht. Beispielweise entsteht bei der Veresterung von Phthalsäure mit n-Nonanol Di-n-nonylphthalat. Besteht dagegen der zur Veresterung eingesetzte Alkohol aus mehreren Isomeren, können beispielsweise bei der Veresterung von Phthalsäure Phthalate, bei denen beide Alkylreste gleich oder verschieden sind, entstehen. So können bei Verwendung des obengenannten Isononanols mit 14 Isomeren 14 Phthalate, bei denen die Alkylgruppen gleich sind, und 91 (14*13/2) Phthalate, bei denen die beiden Alkylgruppen unterschiedlich sind, entstehen. Insgesamt können sich also 105 verschiedene Phthalate, ohne Berücksichtigung von Stereoisomeren, bilden.

Bei Verbindungen wie den Estern der dreiwertigen Trimellit- und der vierwertigen Pyromellitsäure steigt die Isomerenzahl ins Unüberschaubare. Jedes Einzelne der Isomeren müsste vermessen werden, wobei die analytische Auftrennung schon aufwendig und zeitraubend ist, erst recht aber die Herstellung von geeigneten Proben zur Viskositätsmessung.

Überraschend wurde nun gefunden, dass die Viskosität von derartigen Isomerengemischen von Estern mit der Zusammensetzung der zur Veresterung eingesetzten Alkohole bei Polycarbonsäureestern bzw. mit der Zusammensetzung der zur Veresterung von Polyolen eingesetzten Carbonsäuren korreliert.

Die zuvor zitierte Mischungsregel für die Viskosität der Ester ist nicht anwendbar, da nicht die wahre Zusammensetzung der Ester eingeht, sondern nur die Zusammensetzung der zur Veresterung eingesetzten Alkohole bzw. Carbonsäuren. Man erhält so Viskositätskennzahlen der einzelnen Isomeren als Beitrag zur Viskosität des durch Veresterung erhaltenen Estergemisches mit einer vielfachen Anzahl von Isomeren. Enthalten sind in diesen Werten natürlich auch die Einflüsse, die sich durch Wechselwirkung der einzelnen Isomeren in den Estern ergeben.

Es war nicht vorhersehbar, dass ein Estergemisch, welches nur aus Phthalaten besteht, deren Alkylreste gleich sind, eine nahezu gleiche Viskosität aufweist wie ein Phthalatgemisch, in dem neben Phthalaten mit gleichen Alkylresten auch Phthalate mit isomeren Alkylresten vorhanden sind, wenn die Molenbrüche der einzelnen Alkoholkomponenten im Alkoholgemisch, das durch Verseifung des Phthalatgemisches erhalten würde, gleich sind.

Mit anderen Worten: Würde man jedes in einem Alkoholgemisch vorliegende Isomer isolieren, mit Phthalsäure verestern und anschließend die verschiedenen Phthalate im gleichen Verhältnis mischen, wie die Alkohole im Alkoholgemisch vorlagen, so würde man ein Phthalatgemisch mit etwa der gleichen Viskosität erhalten wie dasjenige, das durch direkte Umsetzung des Alkoholgemisches mit Phthalsäure entstanden wäre.

Dieses Ergebnis ist sehr überraschend, weil bei der direkten Veresterung des Alkoholgemisches mit Phthalsäure eine, wie bereits oben ausgeführt wurde, wesentlich höhere Anzahl von Phthalatisomeren entstehen kann, als bei der Veresterung der Einzelkomponenten mit anschließender Mischung im vorgegebenen Verhältnis. Es kommt also nicht auf die tatsächliche Anzahl der Phthalatisomeren im Phthalatgemisch, sondern auf die Isomerenzusammensetzung des zugrundeliegenden Alkoholgemisches an.

Gegenstand der vorliegenden Erfindung ist daher ein Verfahren zur Herstellung von Gemischen isomerer Phthalsäuredialkylester mit einer bestimmten Viskosität durch Verestern von Phthalsäure oder Phthalsäureanhydrid mit einem Gemisch von isomeren Alkylalkoholen, mit einer bestimmten Anzahl an Kohlenstoffatomen, dadurch gekennzeichnet, dass die Viskosität des Phthalsäureestergemisches durch die Zusammensetzung der isomeren Alkylalkohole und deren Viskositätskennzahlen gemäß Formel I

$$\ln(\eta) = \sum x_i * \ln(\eta_i) \qquad \text{Gleichung} \quad (I)$$

mit

$\eta =$ Viskosität des Phthalsäuredialkylestergemisches

$x_i =$ Molenbruch eines isomerenreinen Alkohols

$\eta_i$ = Viskositätskennzahl eines isomerenreinen Alkohols in einem Phthalsäuredialkylestergemisch

eingestellt wird.

Die Viskositäten der Gemische sind von der Anzahl der Kohlenstoffatome der zur Veresterung eingesetzten isomeren Alkylalkohole abhängig. Erfindungsgemäß sind die folgenden Viskositäten bzw. Vorzugsbereiche.

| Anzahl Kohlenstoffatome des isomeren Alkylalkohols | Viskosität des Phthalsäuredialkylestergemischs in mPa*s (20 °C) | | |
|---|---|---|---|
| | erfindungsgemäß | bevorzugt | besonders bevorzugt |
| 4 | 19 bis 44 | 19 bis 44 | 19 bis 44 |
| 5 | 24 bis 50 | 24 bis 50 | 24 bis 50 |
| 6 | 28 bis 80 | 28 bis 70 | 28 bis 60 |
| 7 | 33 bis 100 | 33 bis 70 | 33 bis 65 |
| 8 | 39 bis 130 | 39 bis 110 | 39 bis 100 |
| 9 | 45 bis 200 | 45 bis 165 | 45 bis 120 |
| 10 | 52 bis 400 | 52 bis 330 | 52 bis 200 |
| 11 | 61 bis 400 | 61 bis 380 | 61 bis 350 |
| 12 | 66 bis 400 | 66 bis 380 | 66 bis 350 |
| 13 | 70 bis 400 | 70 bis 380 | 70 bis 350 |
| 14 | 74 bis 400 | 74 bis 380 | 74 bis 350 |

Erfolgt die Herstellung eines Gemisches von isomeren Phthalsäuredialkylestern mit einer bestimmten Viskosität $\eta$ durch Mischen von zwei Phthalsäuredialkylestergemischen, wobei im Grenzfall anstelle der Gemische auch isomerenreine Phthalsäureester eingesetzt werden können, ist es möglich, den Mischungsanteil der beiden Komponenten gemäß Formel II zu berechnen, wenn die Molenbrüche der den Phthalatgemischen zugrundeliegenden isomeren Alkohole bekannt sind.

$$\ln (\eta) = a\sum x_{i1} * \ln (\eta_i) + (1- a) \sum x_{i2} * \ln (\eta_i) \quad \text{Gleichung (II)}$$

mit

$\eta$ = Viskosität des Phthalsäuredialkylestergemisches nach Mischen der beiden Komponenten

$x_{i1}$ = Molenbruch eines Isomeren des Alkoholgemisches 1, das bei der Verseifung des Phthalatgemisches 1 entstünde

$x_{i2}$ = Molenbruch eines Isomeren des Alkoholgemisches 2, das bei der Verseifung des Phthalatgemisches 2 entstünde

$\eta_i$ = Viskossitätskennzahl eines Alkoholisomeren

$a$ = relativer Mischungsanteil des Phthalatgemisches 1 im Zweikomponentengemisch

$(1- a)$ = relativer Mischungsanteil des Phthalatgemisches 2 im Zweikomponentengemisch

$(a/(1-a)$ = Mischungsverhältnis der beiden Komponenten)

Analog der Formel II können Berechnungsvorschriften für die Mischung von mehr als zwei Phthalgemischen aufgestellt werden. Für die Viskosität eines Phthalatgemisches, hergestellt aus drei Phthalatgemischen, gilt beispielsweise

$$\ln (\eta) = a\sum x_{i1} * \ln (\eta_i) + b \sum x_{i2} * \ln (\eta_i) +c \sum x_{i3} * \ln (\eta_a) \quad \text{Gleichung III}$$

mit a + b + c = 1

Dabei sind a, b und c die relativen Mischungsanteile der drei Komponenten im Gesamtgemisch.

Weiterer Gegenstand der vorliegenden Erfindung ist daher ein Verfahren zur Herstellung von Gemischen von isomeren Phthalsäuredialkylestern mit einer bestimmten Viskosität, dadurch gekennzeichnet, dass das Gemisch der isomeren Phthalsäuredialkylester eine Viskosität und Zusammensetzung gemäß Formel IV

$$\ln(\eta) = \sum_{j=1}^{j=n} \sum_{i=1}^{i=m} a_j \; x_{ij} \; \ln(\eta_i) \qquad \text{Gleichung (IV)}$$

mit

$$\sum_{j=1}^{n} a_j = 1 \qquad 0 \le a_j \le 1,$$

n = Anzahl der Mischungskomponenten,

m = Anzahl der dem Endgemisch zugrundeliegenden Alkoholisomere,

$\eta$ = Viskosität des Phthalsäuredialkylestergemisches nach Mischen der Komponenten,

$x_{ij}$ = Molenbruch eines bestimmten Isomeren i im Alkoholgemisch j, das bei der Verseifung des Phthalatgemisches j entstünde,

$\eta_i$ = Viskositätskennzahl eines bestimmten Alkoholisomeren i und

$a_j$ = Mischungsanteil (Massenanteil) einer Komponente j (Phthalatgemisch) im Endprodukt,

aufweist (Gleichungen II und III sind Sonderfälle der allgemein anwendbaren Gleichung IV) und durch Mischen von isomerenreinen Phthalsäuredialkylestern und/oder Phthalsäuredialkylestergemischen, wobei die Alkylester eine gleiche C-Zahl besitzen und eine der Viskosität entsprechende C-Zahl aufweisen, hergestellt wird.

Zur Kettenlänge, Viskosität und deren Vorzugsbereiche gilt das bereits ausgeführte.

Die Gleichungen II bis IV sind auch anwendbar für die Berechnung der Mischungsanteile, wenn ein Alkoholgemisch, hergestellt aus zwei oder mehreren Alkoholgemischen (Komponenten) mit unterschiedlicher Isomerenzusammensetzung (Art der Isomere sowie Mengenanteil), für die Herstellung eines Phthalatgemisches mit vorgegebener Viskosität bereitgestellt werden soll.

Die Phthalsäureisononylester, hergestellt aus Alkylalkoholen mit 9 Kohlenstoffatomen können gewonnen werden, indem als Gemisch von isomeren Alkylalkoholen, ein Gemisch von Nonanolen eingesetzt wird, das durch Mischen eines isomerenreinen Nonanols oder eines Nonanolgemisches mit n-Nonanol hergestellt wurde. Alternativ ist es möglich, als Gemisch von isomeren Alkylalkoholen ein Gemisch von Nonanolen einzusetzen, das durch Mischen eines isomerenreinen Nonanols oder eines Nonanolgemisches mit 3,5,5-Trimethylhexanol hergestellt worden ist, einzusetzen.

Im Falle der Mischung von Phthalsäureestern, z. B. Phthalsäuredinonylester, kann ein isomerenreiner Phthalsäuredinonylester oder ein Phthalsäuredinonylestergemisch mit Phthalsäuredi(3,5,5-trimethylhexanol) oder Phthalsäuredi(isononyl)ester gemischt werden.

Durch geeignetes Abmischen der Hilfskomponenten lassen sich alle Produkte herstellen, deren Viskosität zwischen diesen Grenzen liegen. Mit Hilfe der Alkohole n-Nonanol oder 3,5,5-Trimethylhexanol können beispielsweise DINP-Qualitäten mit Viskositäten im Bereich ~ 45 bis ~ 110 mPa*s eingestellt werden, wobei der erste Fall ein Produkt mit guter Kälteflexibilisierender Wirkung ergibt, der zweite Fall ein Produkt mit weniger guten Kälteflexibilisierenden Wirkung, aber mit erhöhtem elektrischen Widerstand.

Die Phthalsäureestergemische sind durch die einfach einstellbare Viskosität sehr flexibel einsetzbar und können als Weichmacher für Kunststoffe, insbesondere PVC, als Hydraulikflüssigkeit, als Schmiermittel oder als Schmiermittelkomponente verwendet werden.

**Ermittlung der Viskositätsbeiträge der einzelnen Komponenten**

Zum Einen wird man selten sämtliche Isomeren einschließlich aller Spurenbestandteile analysieren können oder wollen wegen des sonst wieder dramatisch ansteigenden Aufwands. In diesem Fall geht man so vor, dass man den unbekannten Rest zusammenfasst und als Scheinkomponente behandelt. Ist dieser unbekannte Rest nicht zu groß, zum Beispiel kleiner 10 % oder besser kleiner 5 %, ist der Fehler nicht unzulässig groß. Eine Spannbreite von +/- 2 mPa*s für das Endprodukt schließt zum Beispiel viele Messungen mit unbekannten Resten von einigen % ein.

Zum Anderen wird man gelegentlich Probleme haben mit einer Autokorrelation von Isomeren. Wenn bei der Oxierung von innenständigen n-Octenen beispielsweise 2-Propyl-hexanol entsteht, wird immer in einem gewissen Anteil auch 2-Ethyl-heptanol und 2-Methyl-octanol vorhanden sein, was zu einer hohen Autokorrelation dieser Isomeren führt. Können diese Isomeren auch durch andere Maßnahmen wie Destillation nicht deutlich differenziert werden, fasst man diese autokorrelierten Bestandteile ebenfalls zu einer einzigen Komponente zusammen. In diesem Fall ist die Auswirkung auf die Genauigkeit sehr gering, denn wenn durch die beschriebenen Maßnahmen schon keine Aufhebung der Autokorrelation möglich ist, wird sie in der praktischen Produktion erst recht vorhanden sein. Die Behandlung von zwei oder mehreren solcher Komponenten als eine einzige ist dann völlig unkritisch und gerechtfertigt.

Es ist überraschend, dass es zur Bestimmung der fiktiven Viskositätsbeiträgen nicht notwendig ist, alle Komponenten zu trennen. Man kann im Fall von Isononanol sogar bis zu drei bis vier Komponenten zusammenfassen, und bleibt immer noch in der erlaubten Spezifikationsbreite. Andererseits kann die Genauigkeit mit entsprechend hohem Aufwand beliebig verbessert werden, wenn besonders hohe Anforderungen hierzu zwingen. Dieser Aufwand ist aber nur ein einziges Mal notwendig. In der Praxis wird man einen Kompromiss zwischen Aufwand und Genauigkeit suchen.

Ist die Ermittlung der Grunddaten einmal erfolgt, ergibt sich auch eine Lösung für die Einhaltung der Grenzwerte. Da der Einfluss der Komponenten jetzt bekannt ist, kann schon in der Herstellung Änderungen der Zusammensetzung und damit der zu erwartenden Eigenschaften begegnet werden. Wichtig ist nicht die exakte Konstanthaltung der Zusammensetzung der Produkte selbst, die wie erläutert technisch gar nicht oder nur mit unvertretbarem Aufwand möglich ist. Wichtig ist vielmehr die Konstanz der anwendungstechnischen Eigenschaften des Endprodukts. In der Praxis geht man so vor, dass man Komponenten, Isomerengemische oder definierte Alkohole bereithält, die deutlich vom gewünschten Wert abweichen.

Als Isomer werden im Folgenden Verbindungen gleicher Summenformel, aber unterschiedlicher Struktur bezeichnet. Isomeren-rein bedeutet, dass der Ester bzw. der Alkohol nur aus Verbindungen eines Isomeren (Stereoisomere werden in diesem Zusammenhang als ein Isomer angesehen) besteht, wobei übliche und/oder technisch bedingte Verunreinigungen anderer Isomere nicht berücksichtigt werden.

Mit den erfindungsgemäß hergestellten Phthalsäureestern bzw. dem Verfahren zu deren Herstellung ist eine schnelle und automatisierbare Kontrolle der Produktion schon über die Vorprodukte möglich, z. B. der Herstellung von Isononanol durch Hydroformylierung von komplexen Octengemischen mit anschließender Hydrierung der Hydroformylierungsprodukte, wobei bei der Hydrierung der Aldehyde zu den entsprechenden Alkoholen der Isoindex und die Art der Verzweigungen der Isomere erhalten bleibt, und es sind Aussagen über die Eigenschaften der hieraus herzustellenden Endprodukte, im Beispiel DINP-Weichmacher, möglich. Beispielsweise kann die Kontrolle durch einen Prozess-Gaschromatografen, gekoppelt mit automatischer Berechnung der zu erwartenden Viskosität der Weichmacher erfolgen. Auf diese Weise ist ein automatisches Monitoring nicht nur der Produktqualität, sondern schon der anwendungstechnischen Eigenschaft der zu erwartenden Endprodukte, in diesem Fall über die physikalische Größe der Viskosität möglich. Da diese aber systematisch mit anderen anwendungstechnischen Eigenschaften korreliert ist, kann im Prinzip jede wichtige Größe im Rahmen der Spezifikationsgrenzen kontrolliert werden. Es sind hierzu nur die notwendigen Messwerte einmal zu ermitteln, um dann eine laufende Kontrolle durchzuführen.

Praktisch geht man so vor, dass man eine Reihe von unterschiedlichen Gemischen mit möglichst unterschiedlicher Isomerenzusammensetzung herstellt. Dazu kann man die zu Beginn genannte Problematik systematisch zur Problemlösung ausnutzen, indem man z. B. Octengemische verwendet mit unterschiedlicher. Zusammensetzung, zum Beispiel käufliche Olefine wie 1-Octen, 4-Octen, 2-Ethylhexen-1 oder 2,4,4-Trimethylpentenen (Diisobuten). Man kann auch Isomerengemische herstellen durch Dehydratisierung von Alkoholen an sauren Katalysatoren, beispielsweise durch Dehydratisierung von Octanol-2 oder 2-Ethylhexanol. Dimethylhexene lassen sich unter anderem herstellen durch saure Oligomerisierung von 1- und 2-Buten. Alle synthetischen Möglichkeiten zur Herstellung von Olefinen können ausgenutzt werden, um möglichst unterschiedliche Ausgangsolefine zu gewinnen.

Die Olefine werden dann hydroformyliert nach unterschiedlichen Verfahren, zum Beispiel mit Co-Hochdruck, Rh-Hochdruck oder mit ligandmodifizierten Rh- und Co-Katalysatoren. Hierdurch erhält man eine weitere Differenzierung der Isomerenzusammensetzung selbst bei dem gleichen eingesetzten Olefin. Hydrierung liefert dann die gewünschten Alkohole, im Beispiel Isononanole, mit völlig unterschiedlicher Isomerenzusammensetzung. Eine weitere Differenzierung kann erreicht werden durch sorgfältige Destillation unter Gewinnung von Isomerenschnitten mit unnatürlicher Isomerenverteilung. Selbstverständlich kann man auch andere synthetische Methoden zur Herstellung von Alkoholen und Aldehyden ausnutzen.

Es sei erwähnt, dass die Einstellung der Viskosität des Endprodukts schon auf der Ebene des Olefinzulaufs zur Hydroformylierung erfolgen kann. So kann die Eindosierung eines Hilfsstoffes, wie z. B. 1-Octen oder eines gering verzweigten Octengemisch, vorgesehen werden zur Erniedrigung der Viskosität des später hieraus herzustellenden Weichmachers sowie die Zudosierung von Diisobuten oder einem stark verzweigten Octengemisch zur analogen Erhöhung der Viskosität. Die während eines Hydroformylierungsschritts sich jeweils einstellende Isomerenverteilung des Oxierungsverfahrens ist durch die laufende Analytik bekannt und kann auf die erforderliche Dosierung des Hilfsstoffes umgerechnet werden. Damit ist auch die Zusammensetzung der durch die Zudosierung veränderten Isomerenverteilung berechenbar und somit auch wieder die Berechnung der zu erwartenden Viskosität des herzustellenden Weichmachers.

Von den unterschiedlichen Alkoholgemischen wird die Zusammensetzung bestimmt. Dabei ist es hilfreich, aber nicht unbedingt notwendig, dass man die Struktur jedes einzelnen Isomeren kennt. Wichtig ist dagegen, dass die Isomeren unterscheidbar sind und ihr Verhältnis zueinander bestimmt werden kann. Da die Analyse der Isomerenzusammensetzung meistens gaschromatographisch durchgeführt wird, können die Isomeren durch ihre Retentionsindices, möglichst auf zwei unterschiedlichen Säulen bestimmt, unterschieden werden.

Im Folgenden soll die Erfindung am Beispiel von Nonanolgemischen näher erläutert werden.

### Beispiel 1

Bei der Analyse von Nonanolgemischen wird beispielsweise wie folgt vorgegangen:

Die Retentionsindices werden auf n-Alkanole bezogen,
n-Heptanol = 700
n-Octanol = 800
n-Nonanol = 900

Da die Messungen in temperaturprogrammierter Fahrweise (siehe Analysenbedingungen) durchgeführt werden, werden die Retentionsindices ($R_i$) der Nonanolisomeren mit Retentionszeiten (RT) zwischen denen von n-Octanol und n-Nonanol nach folgender Formel berechnet:

$$R_i = 800 + (RT_i - RT_{n\text{-}Octanol}) : (RT_{n\text{-}Nonanol} - RT_{n\text{-}Octanol}) * 100$$

### Analysenbedingungen

60 m lange Kapillarsäulen
Stationäre Phasen: polare Säule Polyethylenglykol 20M unpolare Säule 95 % Polydimethylsiloxan/ 5 % Polydiphenylsiloxan
Trägergas : Helium
Gasfluss : 1,5 ml/min
Anfangstemperatur : 60 °C
Endtemperatur : 220 °C
Aufheizrate. 2 °C/min

In Tabelle 1 ist die Analyse eines Isononanolgemisches aufgelistet. Dabei können, wie Analysen einiger ausgewählter Gemische zeigten, Flächen-% mit genügend großer Genauigkeit gleich Gewichtsprozenten gesetzt werden.

Tabelle 1

| Bezeichnung | Ret.Zeit | netto Ret.Zeit. | "Ret. Index" | Siedepunkt | Flächen- % | Gewichts-% |
|---|---|---|---|---|---|---|
| | min | min | eigen | °C | | |
| | | | | | | |
| n-Heptanol | 24,59 | 21,45 | 700 | | | |
| n-Oktanol | 30,7 | 27,56 | 800 | 194,4 | | |
| (4-M-iso-pr-pentanol) | 29,45 | 26,31 | 779 | | 0,3 | 0,3 |

(fortgesetzt)

| Bezeichnung | Ret.Zeit | netto Ret.Zeit. | "Ret. Index" | Siedepunkt | Flächen- % | Gewichts-% |
|---|---|---|---|---|---|---|
| A-2-E-4-M-hexanol | 29,54 | 26,40 | 781 | | 1,5 | 1,5 |
| B-2-E-4-M-hexanol | 30,00 | 26,86 | 788 | | 1,5 | 1,5 |
| A-2,5-DM-heptanol | 30,83 | 27,69 | 802 | | 5,2 | 5,2 |
| B-2,5-DM-heptanol | 30,92 | 27,78 | 804 | | 5,4 | 5,4 |
| A-2-Pr-3-M-pentanol | 31,02 | 27,88 | 805 | | 0,31 | 0,31 |
| X A | 31,14 | 28,00 | 807 | | 0,09 | 0,09 |
| B-2-Pr-3-M-pentanol | 31,405 | 28,26 | 812 | | 0,35 | 0,35 |
| A-2,3,4-TM-hexanol | 32,79 | 29,65 | 835 | | 0,44 | 0,44 |
| 2-Pr-hexanol | 31,54 | 28,40 | 814 | | 1,9 | 1,9 |
| A-2,3-DM-heptanol | 32,125 | 28,98 | 824 | | 1,1 | 1,1 |
| 3-E-4-M-hexanol | 33,5 | 30,36 | 846 | | 4,4 | 3,9 |
| B-2,3-DM-heptanol | 32,7 | 29,56 | 833 | | 1,5 | 1,5 |
| 2-E-heptanol | 32,46 | 29,32 | 829 | | 2,2 | 2,2 |
| 2-M-octanol | 33,06 | 29,92 | 839 | | 4,2 | 4,2 |
| 3-E-heptanol | 33,66 | 30,52 | 849 | | 8,1 | 8,1 |
| A-4,5-DM-heptanol | 34,63 | 31,49 | 865 | | 7,1 | 7,1 |
| B-4,5-DM-heptanol | 34,74 | 31,60 | 867 | | 8,9 | 8,9 |
| 4-M-octanol | 34,12 | 30,98 | 857 | | 19,5 | 19,5 |
| 6-M-octanol | 34,91 | 31,77 | 870 | | 18,7 | 18,7 |
| n-Nonanol | 36,74 | 33,60 | 900 | 213,5 | 7,3 | 7,3 |

**Abkürzungen**

A und B = Diastereomere, die an einer Säule mit nicht optisch aktiven Füllmaterial gaschromatisch unterscheidbar sind.
E = Ethyl-
M = Methyl-
DM = Dimethyl-
Pr = Propyl-
X = unbekannte Verbindung

Von den unterschiedlichen Alkoholgemischen werden dann die Ester hergestellt und wichtige anwendungstechnische Daten bestimmt, vor allem die Viskosität. Sind dagegen Phthalatgemische vorhanden, kann deren Viskosität bestimmt und aus ihnen durch Verseifung das zugrundeliegende Alkoholgemisch gewonnen und analysiert werden. Man wendet dann die Mischungsregel für die Viskosität an, setzt aber an Stelle der nicht zugänglichen Anteile der Esterisomeren die viel einfachere und gut zu bestimmende Isomerenverteilung der Alkohole ein und unterwirft die Datensätze einer nichtlinearen Regressionsrechnung. Man erhält dann als Schätzgröße die fiktiven Viskositätsbeiträge der einzelnen Komponenten.

Dieses Vorgehen wird für Nonylphthalatgemische durchgeführt. In den Tabellen 2 a-f sind die Isomerenverteilungen von 52 Isononanolgemischen und die Viskositäten der daraus hergestellten Nonylphthalate aufgelistet. Diastereomere sind zusammengefasst. Unbekannte Isomere werden summarisch als Scheinkomponente betrachtet. Zusätzlich zu den in Tabelle 1 aufgelisteten Verbindungen enthalten einige Gemische 3,5,5-Trimethylhexanol.

Auf Basis der in den Tabellen 2a-f aufgelisteten Datensätze und der Gleichung 2 werden mit Hilfe des Programmes (DataFit, OAKDALE Engineering, Version 5.1, http:\\www.oakdaleengr.com) für jedes Isononolisomeres ein fiktiver Viskositätsbeitrag (Viskositätskennzahl) (Tabelle 2f., letzte Spalte) erstellt. Dieses Vorgehen kann auch auf andere Nonylphthalatgemische, denen andere oder zusätzlich andere Nonanolisomeren zugrundeliegen, angewandt werden. Die in der Tabelle 2f, letzte Spalte ausgewiesenen Werte für die fiktiven Viskositätsbeiträge können mit steigender Anzahl der Datensätze präzisiert werden.

Tabelle 2a

| Isononylisomere | Probennummer | | | | | | | | |
|---|---|---|---|---|---|---|---|---|---|
| | 1 | 2 | 3 | 4 | 5 | 6 | 7 | 8 | 9 |
| Isononylisomere | Zusammensetzung in Massen-% | | | | | | | | |
| n-Nonanol | 89,5 | 1,2 | 50,8 | 46,1 | 97,5 | 19,2 | 0,4 | 0 | 0 |
| 2-Methyloctanol | 7,9 | 56,2 | 33,5 | 39,0 | 0 | 32,1 | 0 | 0 | 0,1 |
| 2-Ethylheptanol | 1,1 | 24,4 | 9 | 8,8 | 0 | 24,4 | 0,1 | 0 | 0,5 |
| 2-Propylhexanol | 0,3 | 16,6 | 4,7 | 3,8 | 0 | 21,6 | 0 | 0 | 0,8 |
| 4-Methyloctanol | 0,3 | 0,2 | 0,5 | 0,7 | 0 | 0,2 | 1,1 | 6,7 | 0 |
| 2,3-Dimethyl-heptanol | 0 | 0,2 | 0,1 | 0 | 0 | 0,2 | 0 | 0 | 0,1 |
| 3-Ethylheptanol | 0,1 | 0,3 | 0,3 | 0,4 | 0 | 0,8 | 0 | 0,1 | 0 |
| 2-Propyl-3-methyl-pentanol | 0 | 0 | 0 | 0 | 0 | 0 | 0 | 0 | 8,6 |
| 2-Ethyl-4-methyl-hexanol | 0 | 0 | 0 | 0 | 0 | 0,3 | 0 | 0 | 52,0 |
| 2,5-Dimethyl-heptanol | 0 | 0,3 | 0 | 0 | 0 | 0,5 | 0 | 0 | 35,7 |
| 6-Methyloctanol | 0,4 | 0,1 | 0,5 | 0,7 | 0 | 0,2 | 97,5 | 90,1 | 0 |
| 4,5-Dimethyl-heptanol | 0 | 0 | 0 | 0 | 0 | 0,2 | 0,1 | 2,2 | 0 |
| 2,3,4-Trimethyl-hexanol | 0 | 0 | 0 | 0 | 0 | 0 | 0 | 0 | 0,3 |
| 3-Ethyl-4-methyl-hexanol | 0 | 0 | 0 | 0 | 0 | 0 | 0 | 0 | 0 |
| 3,5,5-Trimethyl-hexanol | 0 | 0 | 0 | 0 | 0 | 0 | 0 | 0 | 0 |
| Rest | 0,4 | 0,5 | 0,6 | 0,5 | 2,5 | 0,3 | 0,8 | 0,9 | 1,9 |
| | | | | | | | | | |
| Gemessene Viskosität * | 47,6 | 83,9 | 57,6 | 59,5 | 45,6 | 74,3 | 62,5 | 63,0 | 148,0 |

Tabelle 2b

| Isononylisomere | Probennummer | | | | | | | | |
|---|---|---|---|---|---|---|---|---|---|
| | 10 | 11 | 12 | 13 | 14 | 15 | 16 | 17 | 18 |
| Isononylisomere | Zusammensetzung in Massen-% | | | | | | | | |
| n-Nonanol | 0 | 0 | 0 | 0 | 0,1 | 0 | 0 | 0 | 0 |

(fortgesetzt)

| | Probennummer | | | | | | | | |
|---|---|---|---|---|---|---|---|---|---|
| | 10 | 11 | 12 | 13 | 14 | 15 | 16 | 17 | 18 |
| 2-Methyloctanol | 0 | 0 | 0,2 | 0,1 | 0,3 | 0 | 0 | 1,0 | 3,6 |
| 2-Ethylheptanol | 0 | 2,3 | 0,2 | 0 | 4,4 | 0 | 0,9 | 0,1 | 1,4 |
| 2-Propylhexanol | 0 | 5,5 | 0,1 | 0 | 0,4 | 0 | 0 | 0 | 1,0 |
| 4-Methyloctanol | 12,6 | 0,1 | 16,8 | 23,6 | 5,4 | 1,8 | 0,8 | 37,5 | 13,4 |
| 2,3-Dimethyl-heptanol | 0 | 3,4 | 0,2 | 0 | 7,5 | 0,2 | 0,7 | 0 | 1,2 |
| 3-Ethylheptanol | 0,3 | 0,1 | 0,9 | 1,3 | 3,1 | 0,7 | 0,3 | 5,6 | 8,7 |
| 2-Propyl-3-methyl-pentanol | 0 | 3,2 | 0,1 | 0 | 2,5 | 0 | 0 | 0 | 3,2 |
| 2-Ethyl-4-methyl-hexanol | 0 | 0,8 | 0 | 0 | 7,8 | 0,2 | 0,5 | 0 | 36,2 |
| 2,5-Dimethyl-heptanol | 0,2 | 82,1 | 3,2 | 0 | 8,1 | 0,7 | 0,4 | 0 | 7,1 |
| 6-Methyloctanol | 79,2 | 0 | 67,1 | 61,7 | 1,4 | 1,4 | 0,5 | 28,2 | 1,2 |
| 4,5-Dimethyl-heptanol | 6,2 | 0 | 10,0 | 13,1 | 27,1 | 55,7 | 66,6 | 26,7 | 14,0 |
| 2,3,4-Trimethyl-hexanol | 0 | 2,2 | 0,2 | 0 | 15,8 | 2,8 | 3,6 | 0 | 0,4 |
| 3-Ethyl-4-methyl-hexanol | 0 | 0 | 0,2 | 0,1 | 11,3 | 13,7 | 18,4 | 0,7 | 4,2 |
| 3,5,5-Trimethyl-hexanol | 0 | 0 | 0 | 0 | 0 | 0,3 | 0 | 0 | 0 |
| Rest | 1,5 | 0,3 | 0,8 | 0,1 | 4,8 | 22,5 | 7,3 | 0,2 | 4,4 |
| | | | | | | | | | |
| Gemessene Viskosität * | 65,6 | 125,0 | 66,5 | 65,8 | 140,0 | 116,0 | 110 | 70,6 | 112,0 |

Tabelle 2c

| | Probennummer | | | | | | | | |
|---|---|---|---|---|---|---|---|---|---|
| | 19 | 20 | 21 | 22 | 23 | 24 | 25 | 26 | 27 |
| Isononylisomere | Zusammensetzung in Massen-% | | | | | | | | |
| n-Nonanol | 10,1 | 0 | 7,5 | 0 | 8,6 | 3,6 | 3,4 | 3,4 | 5,8 |
| 2-Methyloctanol | 5,0 | 1,8 | 3,8 | 2,4 | 4,6 | 8,4 | 1,6 | 1,7 | 0,6 |
| 2-Ethylheptanol | 2,6 | 0,2 | 2,2 | 0,3 | 2,4 | 5,3 | 1,1 | 1,2 | 0,3 |
| 2-Propylhexanol | 2,3 | 0 | 1,8 | 0,1 | 2,1 | 5,4 | 0,8 | 0,9 | 0,2 |
| 4-Methyloctanol | 20,7 | 38,2 | 19,7 | 38,2 | 19,6 | 11,3 | 23,8 | 22,4 | 27,7 |
| 2,3-Dimethyl-heptanol | 2,7 | 0,1 | 2,5 | 0,2 | 2,2 | 8,0 | 3,1 | 2,8 | 0,3 |
| 3-Ethylheptanol | 8,5 | 8,3 | 8,2 | 9,9 | 8,1 | 5,0 | 9,3 | 8,9 | 6,7 |
| 2-Propyl-3-methyl-pentanol | 0,7 | 0 | 0,4 | 0 | 0,2 | 1,2 | 0,3 | 0,3 | 0,1 |
| 2-Ethyl-4-methyl-hexanol | 3,1 | 0 | 3,0 | 0 | 2,8 | 9,1 | 2,8 | 2,9 | 0,6 |
| 2,5-Dimethyl-heptanol | 10,3 | 0 | 10,4 | 0,1 | 9,5 | 20,1 | 7,1 | 8,0 | 2,7 |
| 6-Methyloctanol | 20,6 | 19,8 | 19,0 | 14,6 | 20,3 | 7,5 | 15,0 | 15,2 | 21,8 |
| 4,5-Dimethyl-heptanol | 5,8 | 30,1 | 16,0 | 31,5 | 13,5 | 9,5 | 23,6 | 24,2 | 28,1 |
| 2,3,4-Trimethyl-hexanol | 1,7 | 0 | 0,7 | 0 | 0,7 | 1,9 | 1,2 | 1,2 | 0 |
| 3-Ethyl-4-methyl-hexanol | 0,5 | 1,3 | 3,7 | 2,1 | 3,4 | 2,3 | 6,1 | 6,6 | 3,6 |
| 3,5,5-Trimethyl-hexanol | 0,3 | 0 | 0 | 0 | 0 | 0 | 0 | 0 | 0 |

(fortgesetzt)

| Isononylisomere | Zusammensetzung in Massen-% | | | | | | | | |
|---|---|---|---|---|---|---|---|---|---|
| Rest | 5,1 | 0,2 | 1,1 | 0,6 | 2,0 | 1,4 | 0,8 | 0,3 | 1,5 |
| | | | | | | | | | |
| Gemessene Viskosität * | 72,4 | 71,3 | 76,0 | 73,5 | 75,0 | 95,0 | 80,0 | 80,5 | 73,0 |

Tabelle 2d

| | Probennummer | | | | | | | | |
|---|---|---|---|---|---|---|---|---|---|
| | 28 | 29 | 30 | 31 | 32 | 33 | 34 | 35 | 36 |
| Isononylisomere | Zusammensetzung in Massen-% | | | | | | | | |
| n-Nonanol | 2,9 | 6,1 | 4,0 | 0 | 5,2 | 5,4 | 0 | 4,7 | 5,1 |
| 2-Methyloctanol | 8,2 | 3,0 | 1,9 | 3,8 | 3,3 | 3,0 | 4,6 | 3,1 | 2,9 |
| 2-Ethylheptanol | 7,0 | 2,2 | 1,3 | 0,7 | 2,2 | 1,4 | 1,1 | 2,2 | 3,0 |
| 2-Propylhexanol | 5,3 | 2,0 | 1,0 | 0,2 | 2,1 | 1,5 | 0,4 | 2,2 | 1,4 |
| 4-Methyloctanol | 7,7 | 16,6 | 21,2 | 36,3 | 14,7 | 16,6 | 34,4 | 13,8 | 14,7 |
| 2,3-Dimethyl-heptanol | 10,0 | 2,6 | 3,2 | 0,5 | 2,6 | 2,4 | 1,0 | 2,6 | 4,9 |
| 3-Ethylheptanol | 4,8 | 6,8 | 8,3 | 12,6 | 6,2 | 5,6 | 13,6 | 5,9 | 6,1 |
| 2-Propyl-3-methyl-pentanol | 2,2 | 0,9 | 0,4 | 0 | 1,0 | 0 | 0 | 1,1 | 1,3 |
| 2-Ethyl-4-methyl-hexanol | 9,0 | 4,7 | 2,6 | 0 | 5,9 | 2,7 | 0 | 6,5 | 4,5 |
| 2,5-Dimethyl-heptanol | 19,8 | 12,1 | 8,5 | 0,2 | 13,3 | 11,7 | 0,4 | 13,9 | 9,5 |
| 6-Methyloctanol | 6,3 | 15,7 | 14,8 | 9,0 | 13,7 | 18,0 | 7,5 | 12,7 | 12,9 |
| 4,5-Dimethyl-heptanol | 9,0 | 16,9 | 24,6 | 32,3 | 17,8 | 15,6 | 31,3 | 18,4 | 19,4 |
| 2,3,4-Trimethyl-hexanol | 1,8 | 1,2 | 1,2 | 0,1 | 1,0 | 0,6 | 0,2 | 0,9 | 5,7 |
| 3-Ethyl-4-methyl-hexanol | 6,0 | 6,2 | 5,5 | 3,7 | 7,0 | 3,6 | 4,9 | 7,7 | 6,5 |
| 3,5,5-Trimethyl-hexanol | 0 | 0 | 0 | 0 | 0 | 4,0 | 0 | 0 | 0 |
| Rest | 0 | 3,0 | 1,5 | 0,6 | 4,0 | 7,9 | 0,6 | 4,3 | 2,1 |
| | | | | | | | | | |
| Gemessene Viskosität * | 98,0 | 82,6 | 80,8 | 75,2 | 86,7 | 87,1 | 76,4 | 91,7 | 94,7 |

Tabelle 2 e

| | Probennummer | | | | | | | | |
|---|---|---|---|---|---|---|---|---|---|
| | 37 | 38 | 39 | 40 | 41 | 42 | 43 | 44 | 45 |
| Isononylisomere | Zusammensetzung in Massen-% | | | | | | | | |
| n-Nonanol | 4,0 | 9,2 | 0 | 0,4 | 0 | 0 | 0 | 0 | 0 |
| 2-Methyloctanol | 1,5 | 10,2 | 6,9 | 1,6 | 9,7 | 14,0 | 8,2 | 16,0 | 14,9 |
| 2-Ethylheptanol | 1,1 | 5,0 | 2,4 | 2,1 | 4,4 | 6,9 | 16,0 | 8,1 | 11,1 |
| 2-Propylhexanol | 0,9 | 3,8 | 1,1 | 2,6 | 2,7 | 4,8 | 10,5 | 5,5 | 6,5 |
| 4-Methyloctanol | 19,7 | 10,1 | 28,1 | 5,5 | 15,4 | 4,7 | 0,1 | 2,3 | 0,7 |

(fortgesetzt)

| Isononylisomere | Zusammensetzung in Massen-% | | | | | | | | |
|---|---|---|---|---|---|---|---|---|---|
| 2,3-Dimethyl-heptanol | 1,9 | 6,0 | 2,2 | 2,8 | 4,7 | 7,7 | 18,0 | 8,8 | 11,1 |
| 3-Ethylheptanol | 7,5 | 4,2 | 16,6 | 3,0 | 19,0 | 18,0 | 1,7 | 11,0 | 7,0 |
| 2-Propyl-3-methyl-pentanol | 0,4 | 1,5 | 0 | 1,2 | 0,1 | 0,3 | 0,6 | 0,3 | 0,5 |
| 2-Ethyl-4-methyl-hexanol | 2,1 | 6,2 | 0 | 11,6 | 0,1 | 0,2 | 0,3 | 0,2 | 0,3 |
| 2,5-Dimethyl-heptanol | 5,8 | 15,8 | 1,8 | 19,6 | 5,6 | 11,1 | 19,5 | 12,8 | 15,4 |
| 6-Methyloctanol | 14,4 | 7,4 | 2,3 | 3,4 | 0,2 | 0,2 | 0,3 | 0,2 | 0,2 |
| 4,5-Dimethyl-heptanol | 21,5 | 13,7 | 29,2 | 22,4 | 22,1 | 9,5 | 0,3 | 5,2 | 1,7 |
| 2,3,4-Trimethyl-hexanol | 0,8 | 2,5 | 0,7 | 2,0 | 2,5 | 3,2 | 3,5 | 4,4 | 2,9 |
| 3-Ethyl-4-methyl-hexanol | 4,5 | 3,6 | 8,7 | 12,7 | 12,6 | 18,0 | 18,9 | 23,0 | 27,3 |
| 3,5,5-Trimethyl-hexanol | 0,5 | 0 | 0 | 0 | 0 | 0 | 0 | 0 | 0 |
| Rest | 13,4 | 0,8 | 0 | 9,1 | 0,9 | 1,4 | 2,1 | 2,2 | 0,4 |
| | | | | | | | | | |
| Gemessene Viskosität * | 88,0 | 89,7 | 80,2 | 114,0 | 85,9 | 93,4 | 115,0 | 101,0 | 104 |

Tabelle 2f

| | Probennummer | | | | | | | Fiktiver Viskositätsbeitrag der Isomeren |
|---|---|---|---|---|---|---|---|---|
| | 46 | 47 | 48 | 49 | 50 | 51 | 52 | |
| Isononylisomere | Zusammensetzung in Massen-% | | | | | | | |
| n-Nonanol | 0 | 7,8 | 99,4 | 0 | 44,4 | 4,7 | 4,7 | 45,5 |
| 2-Methyloctanol | 0 | 4,2 | 0 | 0 | 2,5 | 2,5 | 2,6 | 72,2 |
| 2-Ethylheptanol | 0 | 2,2 | 0 | 0 | 1,3 | 1,3 | 1,3 | 98,7 |
| 2-Propylhexanol | 0 | 1,9 | 0 | 0 | 1,1 | 1,1 | 1,1 | 98,7 |
| 4-Methyloctanol | 0 | 20,5 | 0 | 0 | 12,3 | 12,3 | 11,5 | 67,2 |
| 2,3-Dimethyl-heptanol | 0 | 2,6 | 0 | 0 | 1,6 | 1,6 | 1,4 | 108,4 |
| 3-Ethylheptanol | 0 | 8,4 | 0 | 0 | 5,0 | 5,0 | 4,5 | 50,5 |
| 2-Propyl-3-methyl-pentanol | 0 | 0,7 | 0 | 0 | 0,4 | 0,4 | 0,4 | 229,5 |
| 2-Ethyl-4-methyl-hexanol | 0 | 2,8 | 0 | 0 | 1,7 | 1,7 | 1,7 | 158,4 |
| 2,5-Dimethyl-heptanol | 0 | 10,2 | 0 | 0 | 6,1 | 6,1 | 6,4 | 120,2 |
| 6-Methyloctanol | 0 | 19,9 | 0 | 0 | 11,9 | 11,9 | 11,1 | 61,1 |
| 4,5-Dimethyl-heptanol | 0 | 13,7 | 0 | 0 | 8,2 | 8,2 | 7,7 | 94,4 |
| 2,3,4-Trimethyl-hexanol | 0 | 0,5 | 0 | 0 | 0,3 | 0,3 | 0,3 | 574,3 |
| 3-Ethyl-4-methyl-hexanol | 0 | 3,5 | 0 | 0 | 2,1 | 2,1 | 1,9 | 120,6 |
| 3,5,5-Trimethyl-hexanol | 97,5 | 0 | 0 | 99,8 | 0 | 39,9 | 42,5 | 111,8 |
| Rest | 2,5 | 1,1 | 0 | 0,2 | 0,9 | 0,7 | 0,9 | (173,1) |
| | | | | | | | | |
| Gemessene Viskosität * | 113,0 | 76,4 | 43,8 | 111,9 | 61,2 | 89,0 | 90,6 | |

**Beispiel 2 Verfahrensvarianten zur Herstellung von Düsononylphthalat (DINP) durch Veresterung von Phthalsäureanhydrid mit einem Gemisch von isomeren Nonanolen:**

Das herzustellende DINP soll eine Viskosität von ca. 78 mPa*s mit den Grenzwerten 75 - 80 mPa*s besitzen. n-Nonanol hat einen Beitrag zur Gesamtviskosität von ca. 43 mPa*s, 3,3,5-Trimethylhexanol von ca. 111 mPa*s.

Steigt die aus der Isomerenverteilung des hergestellten Isononanols berechnete Viskosität des DINP über einen gesetzten Grenzwert, zum Beispiel über 80 mPa*s, dosiert man bei der Veresterung n-Nonanol so zu, dass der gewählte Wert wieder eingestellt wird, im Beispiel also 78 mPa*s. Sinkt die berechnete Viskosität unter einen gewählten Grenzwert, zum Beispiel unter 75 mPa*s, dosiert man bei der Veresterung einfach das viskositätserhöhende 3,5,5-Trimethyl-hexanol zu, bis der gewünschte Wert wieder erreicht ist. Analog zur Beimischung des Alkohols zur Veresterung kann der gleiche Effekt durch Mischen der Ester erreicht werden.

In beiden Fällen kann Gleichung II angewendet werden, die, weil als zweite Komponente ein reines Isomeres eingesetzt wird, folgende Form annimmt:

$$\ln (\eta) = a\sum x_{i1} * \ln (\eta_i) + (1- a) * \ln (\eta_i)$$

mit

$\eta =$  Viskosität des Phthalsäuredialkylestergemisches nach Mischen der beiden Komponenten

$x_{i1} =$  Molenbruch eines Isomeren des Alkoholgemisches 1, das bei der Verseifung des Phthalatgemisches 1 entstünde

$x_{i2} =$  Molenbruch eines Isomeren des Alkoholgemisches 2, das bei der Verseifung des Phthalatgemisches 2 entstünde

$\eta_i =$  Viskossitätskennzahl eines Alkoholisomeren

$a =$  relativer Mischungsanteil des Phthalatgemisches 1 im Zweikomponentengemisch

$(1- a) =$  relativer Mischungsanteil des reinen Phthalats im Zweikomponentengemisch

$(a/(1-a)=$  Mischungsverhältnis der beiden Komponenten)

Mit Hilfe dieser Gleichung können die Mischungsanteile der beiden Komponenten berechnet werden.

n-Nonanol ist kommerziell verfügbar, man kann aber auch käufliches 1-Octen oder billiger innenständige n-Octene hydroformylieren zu einem Isomerengemisch mit niedrigem Viskositätsbeitrag, er muss nur deutlich unter dem gewählten Grenzwert liegen. 3,5,5-Trimethyl-hexanol ist ebenfalls kommerziell erhältlich und kann auch einfach hergestellt werden durch Hydroformylierung von Diisobuten mit nachfolgender Hydrierung, das seinerseits wieder durch Oligomerisierung von Isobuten zugänglich und ein kommerzielles Produkt ist. Schließlich kann man Isomerengemische mit niedrigem und hohem Viskositätsbeitrag vorhalten. In EP 1 029 839 ist gezeigt, wie aus einem Octengemisch ein Isononanol erhalten wird, das in DINP eine Viskosität von 77 mPa*s ergibt. Weiter ist dort beschrieben, dass es möglich ist, das Octengemisch in zwei Fraktionen mit geringerem und höhererem Verzweigungsgrad zu zerlegen. Dabei ergibt die geringer verzweigte Fraktion nach Oxierung, Hydrierung zu den Alkoholen und nachfolgender Veresterung mit Phthalsäureanhydrid ein DINP mit ca. 68 mPa*s, die höher verzweigte Fraktion ein DINP mit ca. 103 mPa*s. Es genügt also für das beschriebene Verfahren, diese beiden Alkoholfraktionen vorzuhalten, die aus dem gleichen Rohstoff stammen wie das erzeugte Isononanol.

In den folgenden Beispielen wird der Zusammenhang zwischen DINP-Gemischen mit unterschiedlicher Isomerenzusammensetzung und deren Voskositäten und anwendungstechnischen Eigenschaften beschrieben.

**Beispiel 3: Herstellung von Phthalsäuredinonylestergemischen (DINP)**

In einem Destillationskolben, der mit Wasserabscheider und Intensivkühler ausgestattet war, wurden 148 g Phthalsäureanhydrid (1 mol), 432 g eines Nonanolgemisches (3 mol, d.h. 50% Überschuss) und 0,5 g Tetra-n-butyltitanat vorgelegt und langsam unter Rühren zum Sieden erhitzt. Die Veresterung verlief bei Normaldruck unter Rückfluß des vorgelegten Alkohols, wobei das anfallende Reaktionswasser am Wasserabscheider sukzessive abgenommen wurde. Es wurde bis zu einer Säurezahl < 0,3 mg KOH/g verestert. Anschließend wurde der Alkohol unter Vakuum abdestilliert. Zur Neutralisation wurde der Rohester auf 80°C abgekühlt und nach Zugabe der entsprechenden Natronlaugemenge unter Normaldruck ca. 30 Minuten gerührt. Danach wurde die organische Phase mehrfach mit Wasser gewaschen und dann die wässrige Phase abgetrennt.

Nun wurde der Ester unter Vakuum auf 180°C aufgeheizt. Bei konstanter Temperatur wurde über ein Tauchrohr VE-Wasser (8 % bezogen auf die Einwaage an Rohester) zugetropft. Nach Ende dieser Wasserdampfdestillation wurde zur Entfernung von Wasserspuren bei 180°C 30 Minuten lang ein Vakuum von 30 mbar angelegt. Das Produkt kühlte nun unter Vakuum bis auf 80°C ab und wurde dann über eine Filternutsche mit Filterpapier und Filterhilfsmittel filtriert.

### Beispiel 4: Bestimmung der Glaspunkte

Von dem so hergestellten Nonylphthalat wird anschließend die dynamische Viskosität bei 20°C nach DIN 53015 und ggf. die Glasübergangstemperatur $T_g$ bestimmt, die ein Maß für die flexibilisierende Wirkung eines Weichmachers ist. Je tiefer die $T_g$ des reinen Weichmachers, desto niedriger liegt bei vorgegebenen Mischungsverhältnis auch die $T_g$ des hiermit hergestellten Weich-PVC und desto höher ist dessen Flexibilität.

Zur Bestimmung von $T_g$ können beispielsweise die Dynamische Differenz-Kalorimetrie (DDK) oder die Torsional Braid Analysis (TBA) verwendet werden. In den hier beschriebenen Fällen wurde auf Grund der höheren Genauigkeit die TBA-Methode durchgeführt. Diese war eine Variante der z.B. in DIN EN ISO 6721 Teil 2 beschriebenen "klassischen" Torsionsschwingungsanalyse (TSA). Bei TBA wurde das zu untersuchende Material (hier der Weichmacher) auf einen in Litzenform geflochtenen und entschlichteten Glasfaser-Roving (Beladung zwischen 18 und 25 Gew. %) aufgebracht. An diesem wurde im Torsionspendel (MYRENNE ATM III) bei Temperaturen zwischen -180 und +100 °C und einer Frequenz von 1 s$^{-1}$ jeweils die Steifigkeit G' und der Verlustmodul G" bestimmt. Aus dem Maximum von G" ließ sich die Glasübergangstemperatur $T_G$ bestimmen.

### Zusammenhang zwischen Glaspunkt und Viskosität bei DINP

Aus verschiedenen Nonanolgemischen wurden, die entsprechenden Diisononylphthalate hergestellt und sowohl die Viskosität als auch die Glasübergangstemperatur des Weichmachers (Phthalats) bestimmt. In der folgenden Tabelle sind die entsprechenden Daten aufgeführt.

| Probe-Bez. | Viskosität bei 20 °C in mPa*s | $T_g$ in °C |
|---|---|---|
| A | 57,6 | -84,1 |
| B | 59,5 | -84,4 |
| C | 62,5 | -86 |
| D | 73 | -82,6 |
| E | 74,3 | -81,9 |
| F | 78 | -81,7 |
| G | 81 | -82 |
| H | 83,9 | -81,3 |
| I | 87,1 | -79,5 |
| J | 89,7 | -79,3 |
| K | 98 | -78,3 |
| L | 110 | -76,7 |
| M | 128 | -73,4 |
| N | 170 | -65,3 |

Es zeigt sich eine praktisch lineare Abhängigkeit von Viskosität und Glasübergangstemperatur. Der Korrelationsfaktor liegt bei 0,99 (Microsoft Excel, statistische Funktion "Korrel").

### Beispiel 5:

Zusammenhang Glaspunkt Weichmacher - Glaspunkt Weich-PVC für DINP

Einige der o.a. zu Phthalaten veresterten Nonanolgemische wurden nach folgender Vorschrift zu weichgemachten PVC-Pressplatten verarbeitet:

700 g Suspensions-PVC mit K-Wert 70 (z.B. VESTOLIT S 7054), wurden mit 300 g des Weichmachers sowie 21 g Pebetal und 2,1 g Bärostab PB 28 F gemischt und bei Temperaturen bis 120 °C zu einem Dryblend verarbeitet (Heizmischer). Danach wurde die Mischung abgekühlt. Zur Erstellung von Walzfellen wurden 250 g des so hergestellten Dryblends auf eine Walze der Fa. Schwabenthan App.-Nr. 1133/0578 (Walzspalt 1,2 mm, Temperatur auf walze 165 °C) gegeben. Nach erfolgter Fellbildung wurde insgesamt weitere 5 min. plastifiziert. Nach Herausnahme des Fells aus dem Walzwerk und Abkühlung wurden aus dem Fell Stücke (ca. 80 g) herausgeschnitten, in eine Schablone 220*220*1 mm eingelegt und in einer hydraulischen Hand-Presse (60 t) der Firma Werner & Pfleiderer wie folgt verpresst: Die Temperatur wurde auf 170 °C eingestellt und die Schablone mit Walzfell zunächst 2 min. bei 50 bar, dann 1 min. bei 100 bar und schließlich nochmals 2 min. bei 180 bar verpresst. Danach wurde der Druck auf 200 bar erhöht und bei diesem Druck auf Raumtemperatur abgekühlt.

| Probe-Bez. | Viskosität | $T_g$ des WM | $T_g$ PVC-P |
|---|---|---|---|
| A | 57,6 | -84,1 | -29,1 |
| B | 59,5 | -84,4 | -27,8 |
| E | 74,3 | -81,9 | -24,4 |
| H | 83,9 | -81,3 | -23,9 |
| K | 98 | -78,3 | -19,6 |

Trägt man die Glaspunkte der reinen Weichmacher gegen die Glaspunkte der hieraus hergestellten Weich-PVC-Platten auf, so ergibt sich auch hier eine eindeutige Korrelation mit einem Koeffizienten von 0,98. Der Korrelationskoeffizient zwischen Viskosität des Weichmachers und der anwendungstechnischen Größe Glastemperatur der Weich-PVC-Platte läßt sich zu 0,98 errechnen (Microsoft Excel, statistische Funktion "Korrel").

Die Beispiele 3-5 belegen, dass die weichmachende Wirkung von DINP mit dessen Viskosität korreliert, die durch die Isomerenzusammensetzung des zugrundeliegenden Nonanolgemisches bestimmt ist.

**Beispiel 6: Zusammenhang zwischen Glaspunkt und Viskosität bei Didecylphthalaten (DIDP)**

Durch Hydroformylierung von 1-Buten, 2-Buten oder Isobuten können die Aldehyde n-Valeraldehyd ("1"), 2-Methylbutanal ("2") und 3-Methylbutanal ("3") entstehen. Je nachdem welche dieser Aldehyde (eventuell auch Mischungen daraus) als Einsatzstoff für die nachfolgende Aldolkondendation und Hydrierung zu den entsprechenden $C_{10}$-Alkoholen verwendet werden, können unterschiedliche Homo- und/oder Coaldolkondensate entstehen.

Diese $C_{10}$-Alkohole wurden, wie für Nonanol beschrieben, zu den entsprechenden Phthalatgemischen (DIDP) umgesetzt und analysiert.

In der folgenden Aufstellung wurden die Viskositäten und Glasübergangstemperaturen für einige aus diesen $C_{10}$-Alkoholen hergestellten Phthalsäureester aufgeführt. Mittels GC und NMR wurden folgende Zusammensetzungen ermittelt, wobei die folgende Kurzschreibweise verwendet wurde:

| Chemische Bezeichnung | Kurzform |
|---|---|
| 2-Isopropyl-5-methyl-hexanol-1 | 3+3 |
| 2-Isopropyl-4-methyl-hexanol-1 (2 Diastereomere) | 2+3 |
| 2-Propyl-5-methyl-hexanol-1/2-Isopropyl-heptanol-1 | 1+3 |
| A-2-Propyl-4-methyl-hexanol-1 (2 Diastereomere) | 1+2 |
| 2-Propylheptanol-1 | 1+1 |

Zusammensetzung und physikalische Daten verschiedener $C_{10}$-Phthalate auf Co-Aldol-Basis:

| Muster-Bez. | 1+1 | 1+2 | 1+3 | 3+2 | 3+3 | Rest | Viskosität in mPa*s | Glaspunkt in °C |
|---|---|---|---|---|---|---|---|---|
| AA | 98,8% | 0 | 0 | 0 | 0 | 1,2% | 118 | -76,8 |
| AB | 14,2% | 85,7% | 0 | 0 | 0 | 0,1% | 201 | -69,9 |
| AC | 7,0% | 0 | 72,3% | 0 | 16,5% | 4,2% | 189 | -69,5 |
| AD | 0 | 0 | 0 | 0 | 99,6% | 0,4% | 313 | -62,7 |
| AE | 0 | 0 | 0 | 70,6% | 29,2% | 0,2% | 368 | -63,3 |
| AF | 29,0% | 57,5% | 3,0% | 3,7% | 5,8% | 1,0 | 176 | -72,3 |

Trägt man hier ebenfalls die Viskosität der $C_{10}$-Phthalate gegen die Glasübergangstemperatur in °C auf, so ergibt sich auch hier eine eindeutige Korrelation (Funktion Korrel, Korrelationsfaktor 0,95).

**Beispiel 6: Einstellung von DINP auf Sollwerte, z.B. 70, 80, 90 mPa*s durch Abmischen**

Isononanol wurde mit n-Nonanol oder 3,5,5-Trimethylhexanol im in der Tabelle angegebenen Verhältnis gemischt und wie oben geschildert, zu den entsprechenden Phthalaten umgesetzt.

| Proben-Bez. | Eingesetzter Alkohol | Viskosität Modell | Viskosität Experiment | Abweichung vom Modell |
|---|---|---|---|---|
| O | Isononanol (INA) | 76,4 | 75,2 | 1,6% |
| P | n-Nonanol (n-C9) | 43,8 | 45,8 | 4,5% |
| Q | 3,5,5-Trimethylhexanol (TMH-ol) | 111,9 | 109 | 2,6% |
| R | INA : n-C9 = 85 :15 | 70 | 69 | 1,4% |
| S | INA: n-C9 = 50 : 50 | 58 | 56,9 | 1,9% |
| T | INA : n-C9 = 75:25 | 66,5 | 64,3 | 3,3 % |
| U | INA: TMH-ol = 80 : 20 | 82,5 | 80,6 | 2,3% |
| V | INA : TMH-ol = 50 : 50 | 92,5 | 89,2 | 3,8% |
| W | INA: TMH-ol = 70:30 | 85,7 | 83,2 | 2,9 % |

Ging man von einer Isomerenverteilung im Isononanol aus, für die das Modell eine Viskosität von 76 mPa*s berechnet (Probe O) und wurde für einen Kunden eine spezielle DINP-Qualität von ca. 70 mPa*s benötigt, so ermittelte das Rechenmodell, dass ca. 15% n-Nonanol vor der Veresterung zugemischt werden müssten.
Die über das Modell vorausgesagten Viskositäten wurden durch die experimentell erhaltenen Werte gut bestätigt (< 5 % Abweichung).

**Patentansprüche**

1. Verfahren zur Herstellung von Gemischen von isomeren Phthalsäuredialkylestern mit einer bestimmten Viskosität durch Verestern von Phthalsäure oder Phthalsäureanhydrid mit einem Gemisch von isomeren Alkylalkoholen mit einer bestimmten Anzahl an Kohlenstoffatomen,
   **dadurch gekennzeichnet,**
   **dass** die Viskosität des Phthalsäureestergemisches durch die Zusammensetzung der isomeren Alkylalkohole und deren Viskositätskennzahlen gemäß Formel I

$$\ln (\eta) = \sum x_i * \ln (\eta_i) \qquad \qquad (I)$$

mit

   $\eta$ = Viskosität des Phthalsäuredialkylestergemisches
   $x_i$ = Molenbruch eines isomerenreinen Alkohols
   $\eta_i$ = Viskositätskennzahl eines isomerenreinen Alkohols in einem Phthalsäuredialkylestergemisch.

eingestellt wird.

2. Verfahren nach Anspruch 1,
   **dadurch gekennzeichnet,**
   **dass** ein Gemisch von isomeren Phthalsäuredialkylestern mit einer Viskosität von 19 bis 44 mPa*s, durch Verestern von Phthalsäure oder Phthalsäureanhydrid mit einem Gemisch von isomeren Alkylalkoholen mit 4 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

3. Verfahren nach Anspruch 1,
   **dadurch gekennzeichnet,**
   **dass** ein Gemisch von isomeren Phthalsäuredialkylestern mit einer Viskosität von 24 bis 50 mPa*s, durch Verestern von Phthalsäure oder Phthalsäureanhydrid mit einem Gemisch von isomeren Alkylalkoholen mit 5 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

4. Verfahren nach Anspruch 1,
   **dadurch gekennzeichnet,**
   **dass** ein Gemisch von isomeren Phthalsäuredialkylestern mit einer Viskosität von 28 bis 80 mPa*s, durch Verestern von Phthalsäure oder Phthalsäureanhydrid mit einem Gemisch von isomeren Alkylalkoholen mit 6 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

5. Verfahren nach Anspruch 1,
   **dadurch gekennzeichnet,**
   **dass** ein Gemisch von isomeren Phthalsäuredialkylestern mit einer Viskosität von 33 bis 100 mPa*s, durch Verestern von Phthalsäure oder Phthalsäureanhydrid mit einem Gemisch von isomeren Alkylalkoholen mit 7 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

6. Verfahren nach Anspruch 1,
   **dadurch gekennzeichnet,**
   **dass** ein Gemisch von isomeren Phthalsäuredialkylestern mit einer Viskosität von 39 bis 130 mPa*s, durch Verestern von Phthalsäure oder Phthalsäureanhydrid mit einem Gemisch von isomeren Alkylalkoholen mit 8 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

7. Verfahren nach Anspruch 1,
   **dadurch gekennzeichnet,**
   **dass** ein Gemisch von isomeren Phthalsäuredialkylestern mit einer Viskosität von 52 bis 400 mPa*s, durch Verestern von Phthalsäure oder Phthalsäureanhydrid mit einem Gemisch von isomeren Alkylalkoholen mit 10 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

8. Verfahren nach Anspruch 1,
   **dadurch gekennzeichnet,**
   **dass** ein Gemisch von isomeren Phthalsäuredialkylestern mit einer Viskosität von 61 bis 400 mPa*s, durch Verestern von Phthalsäure oder Phthalsäureanhydrid mit einem Gemisch von isomeren Alkylalkoholen mit 11 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

9. Verfahren nach Anspruch 1,
   **dadurch gekennzeichnet,**
   **dass** ein Gemisch von isomeren Phthalsäuredialkylestern mit einer Viskosität von 66 bis 400 mPa*s, durch Verestern von Phthalsäure oder Phthalsäureanhydrid mit einem Gemisch von isomeren Alkylalkoholen mit 12 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

10. Verfahren nach Anspruch 1,
    **dadurch gekennzeichnet,**
    **dass** ein Gemisch von isomeren Phthalsäuredialkylestern mit einer Viskosität von 70 bis 400 mPa*s, durch Verestern von Phthalsäure oder Phthalsäureanhydrid mit einem Gemisch von isomeren Alkylalkoholen mit 13 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

**11.** Verfahren nach Anspruch 1,
**dadurch gekennzeichnet,**
**dass** ein Gemisch von isomeren Phthalsäuredialkylestern mit einer Viskosität von 74 bis 400 mPa*s, durch Verestern von Phthalsäure oder Phthalsäureanhydrid mit einem Gemisch von isomeren Alkylalkoholen mit 14 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

**12.** Verfahren nach Anspruch 1,
**dadurch gekennzeichnet,**
**dass** ein Gemisch von isomeren Phthalsäuredialkylestern mit einer Viskosität von 45 bis 200 mPa*s, durch Verestern von Phthalsäure oder Phthalsäureanhydrid mit einem Gemisch von isomeren Alkylalkoholen mit 9 Kohlenstoffatomen hergestellt wird und die Viskosität des Phthalsäureestergemisches durch die Zusammensetzung der isomeren Alkylalkohole und deren Viskositätskennzahlen gemäß Formel I eingestellt wird.

**13.** Verfahren nach Anspruch 12,
**dadurch gekennzeichnet,**
**dass** als Gemisch von isomeren Alkylalkoholen mit 9 Kohlenstoffatomen ein Gemisch von Nonanolen eingesetzt wird, das durch Mischen eines isomerenreinen Nonanols oder eines Nonanolgemisches mit n-Nonanol hergestellt wird.

**14.** Verfahren nach Anspruch 12,
**dadurch gekennzeichnet,**
**dass** als Gemisch von isomeren Alkylalkoholen mit 9 Kohlenstoffatomen ein Gemisch von Nonanolen eingesetzt wird, das durch Mischen eines isomerenreinen Nonanols oder eines Nonanolgemisches mit 3,5,5-Trimethylhexanol hergestellt wird.

**15.** Verfahren zur Herstellung von Gemischen von isomeren Phthalsäuredialkylestern mit einer bestimmten Viskosität,
**dadurch gekennzeichnet,**
**dass** das Gemisch der isomeren Phthalsäuredialkylester eine Viskosität und Zusammensetzung gemäß Formel IV

$$\ln(\eta) = \sum_{j=1}^{j=n} \sum_{i=1}^{i=m} a_j \ x_{ij} \ \ln(\eta_i) \qquad (IV)$$

mit

$$\sum_{j=1}^{n} a_j = 1 \qquad 0 \le a_j \le 1$$

n = Anzahl der Mischungskomponenten
m = Anzahl der dem Endgemisch zugrundeliegenden Alkoholisomere
$\eta$ = Viskosität des Phthalsäuredialkylestergemisches nach Mischen der Komponenten
$x_{ij}$ = Molenbruch eines bestimmten Isomeren i im Alkoholgemisch j, das bei der Verseifung des Phthalatgemisches j entstünde
$\eta_i$ = Viskositätskennzahl eines bestimmten Alkoholisomeren i
$a_j$ = Mischungsanteil (Massenanteil) einer Komponente j (Phthalatgemisch) im Endprodukt,

aufweist und durch Mischen von isomerenreinen Phthalsäuredialkylestern und/oder Phthalsäuredialkylestergemischen, wobei die Alkylester eine gleiche C-Zahl besitzen und eine der Viskosität entsprechende C-Zahl aufweisen, hergestellt wird.

**16.** Verfahren nach Anspruch 15,

**dadurch gekennzeichnet,**
**dass** ein Gemisch von isomeren Phthalsäuredialkylestern mit einer Viskosität von 19 bis 44 mPa*s, durch Mischen von isomerenreinen Phthalsäuredialkylestern und/oder Phthalsäuredialkylestergemischen, wobei die Alkylester 4 Kohlenstoffatome enthalten, hergestellt wird und das Gemisch der isomeren Phthalsäuredialkylester eine Viskosität und Zusammensetzung gemäß Formel IV aufweist.

**17.** Verfahren nach Anspruch 15,
**dadurch gekennzeichnet,**
**dass** ein Gemisch von isomeren Phthalsäuredialkylestern mit einer Viskosität von 24 bis 50 mPa*s, durch Mischen von isomerenreinen Phthalsäuredialkylestern und/oder Phthalsäuredialkylestergemischen, wobei die Alkylester 5 Kohlenstoffatome enthalten, hergestellt wird und das Gemisch der isomeren Phthalsäuredialkylester eine Viskosität und Zusammensetzung gemäß Formel IV aufweist.

**18.** Verfahren nach Anspruch 15,
**dadurch gekennzeichnet,**
**dass** ein Gemisch von isomeren Phthalsäuredialkylestern mit einer Viskosität von 28 bis 80 mPa*s, durch Mischen von isomerenreinen Phthalsäuredialkylestern und/oder Phthalsäuredialkylestergemischen, wobei die Alkylester 6 Kohlenstoffatome enthalten, hergestellt wird und das Gemisch der isomeren Phthalsäuredialkylester eine Viskosität und Zusammensetzung gemäß Formel IV aufweist.

**19.** Verfahren nach Anspruch 15,
**dadurch gekennzeichnet,**
**dass** ein Gemisch von isomeren Phthalsäuredialkylestern mit einer Viskosität von 33 bis 100 mPa*s, durch Mischen von isomerenreinen Phthalsäuredialkylestern und/oder Phthalsäuredialkylestergemischen, wobei die Alkylester 7 Kohlenstoffatome enthalten, hergestellt wird und das Gemisch der isomeren Phthalsäuredialkylester eine Viskosität und Zusammensetzung gemäß Formel IV aufweist.

**20.** Verfahren nach Anspruch 15,
**dadurch gekennzeichnet,**
**dass** ein Gemisch von isomeren Phthalsäuredialkylestern mit einer Viskosität von 39 bis 130 mPa*s, durch Mischen von isomerenreinen Phthalsäuredialkylestern und/oder Phthalsäuredialkylestergemischen, wobei die Alkylester 8 Kohlenstoffatome enthalten, hergestellt wird und das Gemisch der isomeren Phthalsäuredialkylester eine Viskosität und Zusammensetzung gemäß Formel IV aufweist.

**21.** Verfahren nach Anspruch 15,
**dadurch gekennzeichnet,**
**dass** ein Gemisch von isomeren Phthalsäuredialkylestern mit einer Viskosität von 52 bis 400 mPa*s, durch Mischen von isomerenreinen Phthalsäuredialkylestern und/oder Phthalsäuredialkylestergemischen, wobei die Alkylester 10 Kohlenstoffatome enthalten, hergestellt wird und das Gemisch der isomeren Phthalsäuredialkylester eine Viskosität und Zusammensetzung gemäß Formel IV aufweist.

**22.** Verfahren nach Anspruch 15,
**dadurch gekennzeichnet,**
**dass** ein Gemisch von isomeren Phthalsäuredialkylestern mit einer Viskosität von 61 bis 400 mPa*s, durch Mischen von isomerenreinen Phthalsäuredialkylestern und/oder Phthalsäuredialkylestergemischen, wobei die Alkylester 11 Kohlenstoffatome enthalten, hergestellt wird und das Gemisch der isomeren Phthalsäuredialkylester eine Viskosität und Zusammensetzung gemäß Formel IV aufweist.

**23.** Verfahren nach Anspruch 15,
**dadurch gekennzeichnet,**
**dass** ein Gemisch von isomeren Phthalsäuredialkylestern mit einer Viskosität von 66 bis 400 mPa*s, durch Mischen von isomerenreinen Phthalsäuredialkylestern und/oder Phthalsäuredialkylestergemischen, wobei die Alkylester 12 Kohlenstoffatome enthalten, hergestellt wird und das Gemisch der isomeren Phthalsäuredialkylester eine Viskosität und Zusammensetzung gemäß Formel IV aufweist.

**24.** Verfahren nach Anspruch 15,
**dadurch gekennzeichnet,**
**dass** ein Gemisch von isomeren Phthalsäuredialkylestern mit einer Viskosität von 70 bis 400 mPa*s, durch Mischen von isomerenreinen Phthalsäuredialkylestern und/oder Phthalsäuredialkylestergemischen, wobei die Alkylester 13 Kohlenstoffatome enthalten, hergestellt wird und das Gemisch der isomeren Phthalsäuredialkylester eine Viskosität und Zusammensetzung gemäß Formel IV aufweist.

**25.** Verfahren nach Anspruch 15,
**dadurch gekennzeichnet,**
**dass** ein Gemisch von isomeren Phthalsäuredialkylestern mit einer Viskosität von 74 bis 400 mPa*s, durch Mischen von isomerenreinen Phthalsäuredialkylestern und/oder Phthalsäuredialkylestergemischen, wobei, die Alkylester 14 Kohlenstoffatome enthalten, hergestellt wird und das Gemisch der isomeren Phthalsäuredialkylester eine Viskosität und Zusammensetzung gemäß Formel IV aufweist.

**26.** Verfahren nach Anspruch 15,
**dadurch gekennzeichnet,**
**dass** ein Gemisch von isomeren Phthalsäuredialkylestern mit einer Viskosität von 45 bis 200 mPa*s, durch Mischen von isomerenreinen Phthalsäuredialkylestern und/oder Phthalsäuredialkylestergemischen, wobei die Alkylester 9 Kohlenstoffatome enthalten, hergestellt wird und das Gemisch der isomeren Phthalsäuredialkylester eine Viskosität und Zusammensetzung gemäß Formel IV aufweist.

**Claims**

**1.** Process for preparing mixtures of isomeric dialkyl phthalates with a particular viscosity by esterifying phthalic acid or phthalic anhydride with a mixture of isomeric alkyl alcohols having a particular number of carbon atoms,
**characterized in that**
the viscosity of the phthalic ester mixture is adjusted via the composition of the isomeric alkyl alcohols and their viscosity parameters in accordance with formula I.

$$\ln(\eta) = \Sigma \ x_i * \ln(\eta_i) \qquad\qquad (I)$$

where

$\eta$ = viscosity of dialkyl phthalate mixture
$x_i$ = molar fraction of an isomerically pure alcohol
$\eta_i$ = viscosity parameter of an isomerically pure alcohol in a dialkyl phthalate mixture.

**2.** Process according to Claim 1,
**characterized in that**
a mixture of isomeric dialkyl phthalates with a viscosity of from 19 to 44 mPa*s is prepared by esterifying phthalic acid or phthalic anhydride with a mixture of isomeric alkyl alcohols having 4 carbon atoms and the viscosity of the phthalic ester mixture is adjusted via the composition of the isomeric alkyl alcohols and their viscosity parameters in acccordance with formula I.

**3.** Process according to Claim 1,
**characterized in that**
a mixture of isomeric dialkyl phthalates with a viscosity of from 24 to 50 mPa*s is prepared by esterifying phthalic acid or phthalic anhydride with a mixture of isomeric alkyl alcohols having 5 carbon atoms and the viscosity of the phthalic ester mixture is adjusted via the composition of the isomeric alkyl alcohols and their viscosity parameters in accordance with formula I.

**4.** Process according to Claim 1,
**characterized in that**
a mixture of isomeric dialkyl phthalates with a viscosity of from 28 to 80 mPa*s is prepared by esterifying phthalic acid or phthalic anhydride with a mixture of isomeric alkyl alcohols having 6 carbon atoms and the viscosity of the phthalic ester mixture is adjusted via the composition of the isomeric alkyl alcohols and their viscosity parameters in accordance with formula I.

**5.** Process according to Claim 1,
**characterized in that**
a mixture of isomeric dialkyl phthalates with a viscosity of from 33 to 100 mPa*s is prepared by esterifying phthalic acid or phthalic anhydride with a mixture of isomeric alkyl alcohols having 7 carbon atoms and the viscosity of the phthalic ester mixture is adjusted via the composition of the isomeric alkyl alcohols and their viscosity parameters in accordance with formula I.

**6.** Process according to Claim 1,
**characterized in that**
a mixture of isomeric dialkyl phthalates with a viscosity of from 39 to 130 mPa*s is prepared by esterifying phthalic acid or phthalic anhydride with a mixture of isomeric alkyl alcohols having 8 carbon atoms and the viscosity of the phthalic ester mixture is adjusted via the composition of the isomeric alkyl alcohols and their viscosity parameters in accordance with formula I.

**7.** Process according to Claim 1,
**characterized in that**
a mixture of isomeric dialkyl phthalates with a viscosity of from 52 to 400 mPa*s is prepared by esterifying phthalic acid or phthalic anhydride with a mixture of isomeric alkyl alcohols having 10 carbon atoms and the viscosity of the phthalic ester mixture is adjusted via the composition of the isomeric alkyl alcohols and their viscosity parameters in accordance with formula I.

**8.** Process according to Claim 1,
**characterized in that**
a mixture of isomeric dialkyl phthalates with a viscosity of from 61 to 400 mPa*s is prepared by esterifying phthalic acid or phthalic anhydride with a mixture of isomeric alkyl alcohols having 11 carbon atoms and the viscosity of the phthalic ester mixture is adjusted via the composition of the isomeric alkyl alcohols and their viscosity parameters in accordance with formula I.

**9.** Process according to Claim 1,
**characterized in that**
a mixture of isomeric dialkyl phthalates with a viscosity of from 66 to 400 mPa*s is prepared by esterifying phthalic acid or phthalic anhydride with a mixture of isomeric alkyl alcohols having 12 carbon atoms and the viscosity of the phthalic ester mixture is adjusted via the composition of the isomeric alkyl alcohols and their viscosity parameters in accordance with formula I.

**10.** Process according to Claim 1,
**characterized in that**
a mixture of isomeric dialkyl phthalates with a viscosity of from 70 to 400 mPa*s is prepared by esterifying phthalic acid or phthalic anhydride with a mixture of isomeric alkyl alcohols having 13 carbon atoms and the viscosity of the phthalic ester mixture is adjusted via the composition of the isomeric alkyl alcohols and their viscosity parameters in accordance with formula I.

**11.** Process according to Claim 1,
**characterized in that**
a mixture of isomeric dialkyl phthalates with a viscosity of from 74 to 400 mPa*s is prepared by esterifying phthalic acid or phthalic anhydride with a mixture of isomeric alkyl alcohols having 14 carbon atoms and the viscosity of the phthalic ester mixture is adjusted via the composition of the isomeric alkyl alcohols and their viscosity parameters in accordance with formula I.

**12.** Process according to Claim 1,
**characterized in that**
a mixture of isomeric dialkyl phthalates with a viscosity of from 45 to 200 mPa*s is prepared by esterifying phthalic acid or phthalic anhydride with a mixture of isomeric alkyl alcohols having 9 carbon atoms and the viscosity of the phthalic ester mixture is adjusted via the composition of the isomeric alkyl alcohols and their viscosity parameters in accordance with formula I.

**13.** Process according to Claim 12,
**characterized in that**

the mixture of isomeric alkyl alcohols having 9 carbon atoms comprises a mixture of nonanols which is prepared by mixing an isomerically pure nonanol or a nonanol mixture with n-nonanol.

14. Process according to Claim 12,
**characterized in that**
the mixture of isomeric alkyl alcohols having 9 carbon atoms comprises a mixture of nonanols which is prepared by mixing an isomerically pure nonanol or a nonanol mixture with 3,5,5-trimethylhexanol.

15. Process for preparing mixtures of isomeric dialkyl phthalates with a particular viscosity,
**characterized in that**
the mixture of the isomeric dialkyl phthalates has a viscosity and composition in accordance with formula IV

$$\ln(\eta) = \sum_{j=1}^{j=n} \sum_{i=1}^{i=m} a_j \, x_{ij} \, \ln(\eta_i) \qquad (IV)$$

where

$$\sum_{j=1}^{n} a_j = 1 \qquad 0 \leq a_j \leq 1$$

n = number of components in mixture
m = number of alcohol isomers underlying the final mixture
$\eta$ = viscosity of dialkyl phthalate mixture after mixing the components
$x_{ij}$ = molar fraction of a particular isomer i in alcohol mixture j which would result from saponification of phthalate mixture j
$\eta_i$ = viscosity parameter of a particular alcohol isomer i
$a_j$ = mixture content (proportion by weight) of a component j (phthalate mixture) in final product,

and is prepared by mixing isomerically pure dialkyl phthalates and/or dialkyl phthalate mixtures, where the alkyl ester groups have the same carbon number and have a carbon number corresponding to the viscosity.

16. Process according to Claim 15,
**characterized in that**
a mixture of isomeric dialkyl phthalates with a viscosity of from 19 to 44 mPa*s is prepared by mixing isomerically pure dialkyl phthalates and/or dialkyl phthalate mixtures, where the alkyl ester groups contain 4 carbon atoms, and the mixture of isomeric dialkyl phthalates has a viscosity and composition in accordance with formula IV.

17. Process according to Claim 15,
**characterized in that**
a mixture of isomeric dialkyl phthalates with a viscosity of from 24 to 50 mPa*s is prepared by mixing isomerically pure dialkyl phthalates and/or dialkyl phthalate mixtures, where the alkyl ester groups contain 5 carbon atoms and the mixture of the isomeric dialkyl phthalates has a viscosity and composition in accordance with formula IV.

18. Process according to Claim 15,
**characterized in that**
a mixture of isomeric dialkyl phthalates with a viscosity of from 28 to 80 mPa*s is prepared by mixing isomerically pure dialkyl phthalates and/or dialkyl phthalate mixtures, where the alkyl ester groups contain 6 carbon atoms and the mixture of the isomeric dialkyl phthalates has a viscosity and composition in accordance with formula IV.

19. Process according to Claim 15,

**characterized in that**
a mixture of isomeric dialkyl phthalates with a viscosity of from 33 to 100 mPa*s is prepared by mixing isomerically pure dialkyl phthalates and/or dialkyl phthalate mixtures, where the alkyl ester groups contain 7 carbon atoms and the mixture of the isomeric dialkyl phthalates has a viscosity and composition in accordance with formula IV.

20. Process according to Claim 15,
**characterized in that**
a mixture of isomeric dialkyl phthalates with a viscosity of from 39 to 130 mPa*s is prepared by mixing isomerically pure dialkyl phthalates and/or dialkyl phthalate mixtures, where the alkyl ester groups contain 8 carbon atoms and the mixture of the isomeric dialkyl phthalates has a viscosity and composition in accordance with formula IV.

21. Process according to Claim 15,
**characterized in that**
a mixture of isomeric dialkyl phthalates with a viscosity of from 52 to 400 mPa*s is prepared by mixing isomerically pure dialkyl phthalates and/or dialkyl phthalate mixtures, where the alkyl ester groups contain 10 carbon atoms and the mixture of the isomeric dialkyl phthalates has a viscosity and composition in accordance with formula IV.

22. Process according to Claim 15,
**characterized in that**
a mixture of isomeric dialkyl phthalates with a viscosity of from 61 to 400 mPa*s is prepared by mixing isomerically pure dialkyl phthalates and/or dialkyl phthalate mixtures, where the alkyl ester groups contain 11 carbon atoms and the mixture of the isomeric dialkyl phthalates has a viscosity and composition in accordance with formula IV.

23. Process according to Claim 15,
**characterized in that**
a mixture of isomeric dialkyl phthalates with a viscosity of from 66 to 400 mPa*s is prepared by mixing isomerically pure dialkyl phthalates and/or dialkyl phthalate mixtures, where the alkyl ester groups contain 12 carbon atoms and the mixture of the isomeric dialkyl phthalates has a viscosity and composition in accordance with formula IV.

24. Process according to Claim 15,
**characterized in that**
a mixture of isomeric dialkyl phthalates with a viscosity of from 70 to 400 mPa*s is prepared by mixing isomerically pure dialkyl phthalates and/or dialkyl phthalate mixtures, where the alkyl ester groups contain 13 carbon atoms and the mixture of the isomeric dialkyl phthalates has a viscosity and composition in accordance with formula IV.

25. Process according to Claim 15,
**characterized in that**
a mixture of isomeric dialkyl phthalates with a viscosity of from 74 to 400 mPa*s is prepared by mixing isomerically pure dialkyl phthalates and/or dialkyl phthalate mixtures, where the alkyl ester groups contain 14 carbon atoms and the mixture of the isomeric dialkyl phthalates has a viscosity and composition in accordance with formula IV.

26. Process according to Claim 15,
**characterized in that**
a mixture of isomeric dialkyl phthalates with a viscosity of from 45 to 200 mPa*s is prepared by mixing isomerically pure dialkyl phthalates and/or dialkyl phthalate mixtures, where the alkyl ester groups contain 9 carbon atoms and the mixture of the isomeric dialkyl phthalates has a viscosity and composition in accordance with formula IV.

**Revendications**

1. Procédé de fabrication de mélanges de dialkylesters d'acide phtalique isoméres présentant une certaine viscosité par estérification d'acide phtalique ou d'anhydride d'acide phtalique et d'un mélange d'alcools d'alkyle isomères présentant un certain nombre d'atomes de carbone,
**caractérisé en ce que**
la viscosité du mélange d'esters d'acide phtalique est ajustée par la composition des alcools d'alkyle isomères et de leur indice de viscosité selon la formule I

$$ln(\eta) = \Sigma \; x_i \; * \; ln \; (\eta_i) \qquad (I)$$

où $\eta$ = viscosité du mélange de dialkylesters d'acide phtalique

$x_i$ = fraction molaire d'un alcool purement isomère

$\eta_i$ = indice de viscosité d'un alcool purement isomère dans un mélange de dialkylesters d'acide phtalique.

2. Procédé selon la revendication 1,
   **caractérisé en ce qu'**
   un mélange de dialkylesters d'acide phtalique isomères présentant une viscosité de 19 à 44 mPa*s est produit par estérification d'acide phtalique ou d'anhydride d'acide phtalique avec un mélange d'alcools d'alkyle isomères comportant 4 atomes de carbone et la viscosité du mélange d'esters d'acide phtalique est ajustée par la composition des alcools d'alkyle isomères et leurs indices de viscosité selon la formule I.

3. Procédé selon la revendication 1,
   **caractérisé en ce qu'**
   un mélange de dialkylesters d'acide phtalique isomères présentant une viscosité de 24 à 50. mPa*s, est produit par estérification d'acide phtalique ou d'anhydride d'acide phtalique avec un mélange d'alcools d'alkyle isomères comportant 5 atomes de carbone et la viscosité du mélange d'esters d'acide phtalique est ajustée par la composition des alcools d'alkyle isomères et leurs indices de viscosité selon la formule I.

4. Procédé selon la revendication 1,
   **caractérisé en ce qu'**
   un mélange de dialkylesters d'acide phtalique isomères présentant une viscosité de 28 à 80 mPa*s est produit par estérification d'acide phtalique ou d'anhydride d'acide phtalique avec un mélange d'alcools d'alkyle isomères comportant 6 atomes de carbone et la viscosité du mélange d'esters d'acide phtalique est ajustée par la composition des alcools d'alkyle isomères et leurs indices de viscosité selon la formule I.

5. Procédé selon la revendication 1,
   **caractérisé en ce qu'**
   un mélange de dialkylesters d'acide phtalique isomères présentant une viscosité de 33 à 100 mPa*s est produit par estérification d'acide phtalique ou d'anhydride d'acide phtalique avec un mélange d'alcools d'alkyle isomères comportant 7 atomes de carbone et la viscosité du mélange d'esters d'acide phtalique est ajustée par la composition des alcools d'alkyle isomères et leurs indices de viscosité selon la formule I.

6. Procédé selon la revendication 1,
   **caractérisé en ce qu'**
   un mélange de dialkylesters d'acide phtalique isomères présentant une viscosité de 39 à 130 mPa*s est produit par estérification d'acide phtalique ou d'anhydride d'acide phtalique avec un mélange d'alcools d'alkyle isomères comportant 8 atomes de carbone et la viscosité du mélange d'esters d'acide phtalique est ajustée par la composition des alcools d'alkyle isomères et leurs indices de viscosité selon la formule I.

7. Procédé selon la revendication 1,
   **caractérisé en ce qu'**
   un mélange de dialkylesters d'acide phtalique isomères présentant une viscosité de 52 à 400 mPa*s est produit par estérification d'acide phtalique ou d'anhydride d'acide phtalique avec un mélange d'alcools d'alkyle isomères comportant 10 atomes de carbone et la viscosité du mélange d'esters d'acide phtalique est ajustée par la composition des alcools d'alkyle isomères et leurs indices de viscosité selon la formule I.

8. Procédé selon la revendication 1,
   **caractérisé en ce qu'**
   un mélange de dialkylesters d'acide phtalique isomères présentant une viscosité de 61 à 400 mPa*s est produit par estérification d'acide phtalique ou d'anhydride d'acide phtalique avec un mélange d'alcools d'alkyle isomères comportant 11 atomes de carbone et la viscosité du mélange d'esters d'acide phtalique est ajustée par la composition des alcools d'alkyle isomères et leurs indices de viscosité selon la formule I.

9. Procédé selon la revendication 1,

**caractérisé en ce qu'**
un mélange de dialkylesters d'acide phtalique isomères présentant une viscosité de 66 à 400 mPa*s est produit par estérification d'acide phtalique ou d'anhydride d'acide phtalique avec un mélange d'alcools d'alkyle isomères comportant 12 atomes de carbone et la viscosité du mélange d'esters d'acide phtalique est ajustée par la composition des alcools d'alkyle isomères et leurs indices de viscosité selon la formule I.

10. Procédé selon la revendication 1,
**caractérisé en ce qu'**
un mélange de dialkylesters d'acide phtalique isomères présentant une viscosité de 70 à 400 mPa*s est produit par estérification d'acide phtalique ou d'anhydride d'acide phtalique avec un mélange d'alcools d'alkyle isomères comportant 13 atomes de carbone et la viscosité du mélange d'esters d'acide phtalique est ajustée par la composition des alcools d'alkyle isomères et leurs indices de viscosité selon la formule I.

11. Procédé selon la revendication 1,
**caractérisé en ce qu'**
un mélange de dialkylesters d'acide phtalique isomères présentant une viscosité de 74 à 400 mPa*s est produit par estérification d'acide phtalique ou d'anhydride d'acide phtalique avec un mélange d'alcools d'alkyle isomères comportant 14 atomes de carbone et la viscosité du mélange d'esters d'acide phtalique est ajusté par la composition des alcools d'alkyle isomères et leurs indices de viscosité selon la formule I.

12. Procédé selon la revendication 1,
**caractérisé en ce qu'**
un mélange de dialkylesters d'acide phtalique isomères présentant une viscosité de 45 à 200 mPa*s est produit par estérification d'acide phtalique ou d'anhydride d'acide phtalique avec un mélange d'alcools d'alkyle isomères comportant 9 atomes de carbone et la viscosité du mélange d'esters d'acide phtalique est ajustée par la composition des alcools d'alkyle isomères et leurs indices de viscosité selon la formule I.

13. Procédé selon la revendication 12,
**caractérisé en ce que**
l'on utilise comme mélange d'alcools d'alkyle isomères comportant 9 atomes de carbone, un mélange de nonanols qui est produit par mélange d'un nonanol purement isomère ou d'un mélange de nonanols avec du n-nonanol.

14. Procédé selon la revendication 12,
**caractérisé en ce que**
l'on utilise comme mélange d'alcools d'alkyle isomères comportant 9 atomes de carbone, un mélange de nonanols qui est produit par mélange d'un nonanols purement isomère ou d'un mélange de nonanols avec du 3,5,5-triméthylhexanol.

15. Procédé de production de mélanges de dialkylesters d'acide phtalique isomères présentant une certaine viscosité,
**caractérisé en ce que**
le mélange de dialkylesters d'acide phtalique isomères présente une viscosité et une composition selon la formule IV

$$\ln(\eta) = \sum_{j=1}^{j=n} \sum_{i=1}^{i=m} a_j \, x_{ij} \, \ln(\eta_i) \qquad (IV)$$

avec

$$\sum_{j=1}^{n} a_j = 1 \qquad 0 \le a_j \le 1$$

n = nombre des composants du mélange

m = nombre des isomères d'alcool à la base du mélange final

$\eta$ = viscosité du mélange de dialkylesters d'acide phtalique après mélange des composants

$x_{ij}$ = fraction molaire d'un isomère particulier i dans le mélange d'alcools j qui serait obtenu par saponification du mélange de phtalate

$\eta_i$ = indice de viscosité d'un isomère d'alcool particulier i

$a_j$ = proportion du mélange (proportion en masse) d'un composant j (mélange de phtalate) dans le produit final,

et est produit par mélange de dialkylesters d'acide phtalique purement isomères et/ou de mélanges de dialkylesters d'acide phtalique, l'alkylester présentant un nombre C identique et un nombre C correspondant à la viscosité.

16. Procédé selon la revendication 15,
**caractérisé en ce qu'**
un mélange de dialkylesters d'acide phtalique isomères présentant une viscosité de 19 à 44 mPa*s est produit par mélange de dialkylesters d'acide phtalique purement isomères et/ou de mélanges de dialkylesters d'acide phtalique, l'alkylester contenant 4 atomes de carbone et le mélange de dialkylesters d'acide phtalique isomères présentant une viscosité et une composition selon la formule IV.

17. Procédé selon la revendication 15,
**caractérisé en ce qu'**
un mélange de dialkylesters d'acide phtalique isomères présentant une viscosité de 24 à 50 mPa*s est produit par mélange de dialkylesters d'acide phtalique purement isomères et/ou de mélanges de dialkylesters d'acide phtalique, l'alkylester contenant 5 atomes de carbone et le mélange de dialkylesters d'acide phtalique isomères présentant une viscosité et une composition selon la formule IV.

18. Procédé selon la revendication 15,
**caractérisé en ce qu'**
un mélange de dialkylesters d'acide phtalique isomères présentant une viscosité de 28 à 80 mPa*s est produit par mélange de dialkylesters d'acide phtalique purement isomères et/ou de mélanges de dialkylesters d'acide phtalique, l'alkylester contenant 6 atomes de carbone et le mélange de dialkylesters d'acide phtalique isomères présentant une viscosité et une composition selon la formule IV.

19. Procédé selon la revendication 15,
**caractérisé en ce qu'**
un mélange de dialkylesters d'acide phtalique isomères présentant une viscosité de 33 à 100 mPa*s est produit par mélange de dialkylesters d'acide phtalique purement isomères et/ou de mélanges de dialkylesters d'acide phtalique, l'alkylester contenant 7 atomes de carbone et le mélange de dialkylesters d'acide phtalique isomères présentant une viscosité et une composition selon la formule IV.

20. Procédé selon la revendication 15,
**caractérisé en ce qu'**
un mélange de dialkylesters d'acide phtalique isomères présentant une viscosité de 39 à 130 mPa*s est produit par mélange de dialkylesters d'acide phtalique purement isomères et/ou de mélanges de dialkylesters d'acide phtalique, l'alkylester contenant 8 atomes de carbone et le mélange de dialkylesters d'acide phtalique isomères présentant une viscosité et une composition selon la formule IV.

21. Procédé selon la revendication 15,
**caractérisé en ce qu'**
un mélange de dialkylesters d'acide phtalique isomères présentant une viscosité de 52 à 400 mPa*s est produit par mélange de dialkylesters d'acide phtalique purement isomères et/ou de mélanges de dialkylesters d'acide phtalique, l'alkylester contenant 10 atomes de carbone et le mélange de dialkylesters d'acide phtalique isomères présentant une viscosité et une composition selon la formule IV.

22. Procédé selon la revendication 15,
**caractérisé en ce qu'**
un mélange de dialkylesters d'acide phtalique isomères présentant une viscosité de 61 à 400 mPa*s est produit par mélange de dialkylesters d'acide phtalique purement isomères et/ou de mélanges de dialkylesters d'acide phtalique, l'alkylester contenant 11 atomes de carbone et le mélange de dialkylesters d'acide phtalique isomères présentant une viscosité et une composition selon la formule IV.

**23.** Procédé selon la revendication 15,
**caractérisé en ce qu'**
un mélange de dialkylesters d'acide phtalique isomères présentant une viscosité de 66 à 400 mPa*s est produit par mélange de dialkylesters d'acide phtalique purement isomères et/ou de mélanges de dialkylesters d'acide phtalique, l'alkylester contenant 12 atomes de carbone et le mélange de dialkylesters d'acide phtalique isomères présentant une viscosité et une composition selon la formule IV.

**24.** Procédé selon la revendication 15,
**caractérisé en ce qu'**
un mélange de dialkylesters d'acide phtalique isomères présentant une viscosité de 70 à 400 mPa*s est produit par mélange de dialkylesters d'acide phtalique purement isomères et/ou de mélanges de dialkylesters d'acide phtalique, l'alkylester contenant 13 atomes de carbone et le mélange de dialkylesters d'acide phtalique isomères présentant une viscosité et une composition selon la formule IV.

**25.** Procédé selon la revendication 15,
**caractérisé en ce qu'**
un mélange de dialkylesters d'acide phtalique isomères présentant une viscosité de 74 à 400 mPa*s est produit par mélange de dialkylesters d'acide phtalique purement isomères et/ou de mélanges de dialkylesters d'acide phtalique, l'alkylester contenant 14 atomes de carbone et le mélange de dialkylesters d'acide phtalique isomères présentant une viscosité et une composition selon la formule IV.

**26.** Procédé selon la revendication 15,
**caractérisé en ce qu'**
un mélange de dialkylesters d'acide phtalique isomères présentant une viscosité de 45 à 200 mPa*s est produit par mélange de dialkylesters d'acide phtalique purement isomères et/ou de mélanges de dialkylesters d'acide phtalique, l'alkylester contenant 9 atomes de carbone et le mélange de dialkylesters d'acide phtalique isomères présentant une viscosité et une composition selon la formule IV.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1029839 A

**In der Beschreibung aufgeführte Nicht-Patentliteratur**

- **BRIAN L. WADEY ; LUCIEN THIL ; MO A. KHUDDUS ; HANS REICH.** The Nonyl Phthalate Ester and It's Use in flexible PVC. *Journal of Vinyl Technology,* 1990, vol. 12 (4), 208-211